(19) Europäisches Patentamt
European Patent Office
Office européen des brevets

(11) Publication number: **0 014 476**
**B1**

(12) # EUROPEAN PATENT SPECIFICATION

(45) Date of publication of patent specification: **10.08.83**

(21) Application number: **80100666.9**

(22) Date of filing: **08.02.80**

(51) Int. Cl.³: **C 07 D 471/04, C 12 P 17/18, A 61 K 31/435 //C12N9/80, (C07D471/04, 221/00, 205/00)**

(54) **Optically active acylated cephalosporin analogs, processes for their preparation and pharmaceutical compositions comprising them.**

(30) Priority: **10.02.79 JP 14534/79**
**14.04.79 JP 45897/79**
**19.07.79 JP 92035/79**
**01.11.79 JP 140476/79**
**14.11.79 JP 146489/79**

(43) Date of publication of application:
**20.08.80 Bulletin 80/17**

(45) Publication of the grant of the patent:
**10.08.83 Bulletin 83/32**

(84) Designated Contracting States:
**BE CH DE FR GB IT NL SE**

(56) References cited:
**DE - A - 2 714 880**
**GB - A - 2 017 103**

**Cephalosporins and penicillins' pages 13 and 540**

**Burgers Medicinal Chemistry, 3rd Edition, pages 81—87**

**The file contains technical information submitted after the application was filed and not included in this specification**

(73) Proprietor: **KYOWA HAKKO KOGYO CO., LTD**
**Ohtemachi Bldg. Ohtemachi 1-chome Chiyoda-ku**
**Tokyo (JP)**

(72) Inventor: **Hirata, Tadashi**
**1566-315, Nara-machi, Midori-ku**
**Yokohama-shi, Kanagawa-ken (JP)**
Inventor: **Hashimoto, Yukio**
**3-5-15, Chuorinkan**
**Yamato-shi, Kanagawa-ken (JP)**
Inventor: **Ogasa, Takehiro**
**3-6-6, Asahi-machi**
**Machida-shi, Tokyo (JP)**
Inventor: **Kobayashi, Shigeru**
**71-9, Kiso-machi**
**Machida-shi, Tokyo (JP)**
Inventor: **Matsukuma, Ikuo**
**2273-1, Ikuwa-cho**
**Yokkaichi-shi, Mie-ken (JP)**
Inventor: **Kimura, Kazuo**
**2-1-5, Kyowa-cho**
**Hofu-shi, Yamaguchi-ken (JP)**
Inventor: **Yoshiie, Shigeo**
**2-14-10, Asahi-machi**
**Machida-shi, Tokyo (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 014 476 B1

(72) Inventor: **Sato, Kiyoshi**
**326, Fushimi**
**Shimizu-cho, Sunto-gun Shizuoka-ken (JP)**
Inventor: **Ohasi, Yoichi**
**122-4, Mizukubo**
**Susono-shi, Shizuoka-ken (JP)**
Inventor: **Takasawa, Seigo**
**125-5, Minamiyana**
**Hadano-shi, Kanagawa-ken (JP)**

(74) Representative: **Vossius Vossius Tauchner**
**Heunemann Rauh**
**Siebertstrasse 4 P.O. Box 86 07 67**
**D-8000 München 86 (DE)**

# Optically active acylated cephalosporin analogs, processes for their preparation and pharmaceutical compositions comprising them

The present invention relates to optically active compounds of cephalosporin analogs represented by the general formula (I)

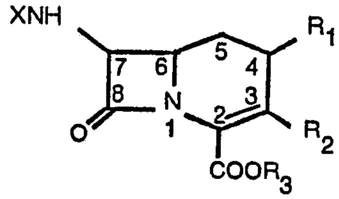

(I)

wherein $R_1$ represents a hydrogen or a lower alkyl group having 1 to 5 carbon atoms, $R_2$ represents a hydrogen or a halogen atom, $R_3$ represents a hydrogen or a protective group of carboxylic acid, X represents an acyl group represented by the formula $X_1CO$ wherein $X_1$ represents the following two groups:

1) a group represented by the general formula

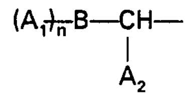

[wherein B represents an unsaturated six membered carbocycle which is selected from cyclohexenyl group, cyclohexadienyl group and phenyl group or a five or six membered heterocycle as defined in Claim 1, $A_1$ represents substituent(s) which is selected from hydroxyl group, a lower alkoxy group having 1 to 4 carbon atoms, a halo group, nitro group, amino group, aminomethyl group, and a methylsulfonamido group, n is a number from 0 to 5, and $A_2$ represents amino group, hydroxyl group, carboxyl group or sulfo group].

2) a group represented by the general formula

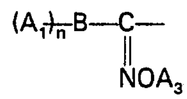

[wherein $A_1$, B and n have the same significance as defined above and $A_3$ represents hydrogen atom, a lower alkyl group having 1 to 6 carbon atoms, a lower alkenyl group having 2 to 6 carbon atoms, a lower alkynyl group having 2 to 6 carbon atoms, a cycloalkyl group having 3 to 6 carbon atoms or an aryl group, those groups being unsubstituted or substituted with suitable substituent(s) which is selected from carboxyl group, cyano group, a halo group, carbamoyl group and a lower alkoxycarbonyl group having 1 to 5 carbon atoms], and the hydrogens at the 6- and 7-positions have cis configuration and pharmaceutically acceptable salts thereof.

A carbacephem compound, which is named according to the nomenclature in J. Am. Chem. Soc. *96,* 7584 (1974), wherein the sulfur atom of cephalosporin is substituted with a carbon atom and which has a substituted methyl group at the 3-position is described in the above reference and J. Med. Chem. *20,* 551 (1977). However, no compound having especially strong antibacterial activity has been reported. In Japanese Published Unexamined Patent Application No. 9296/79 (German Offenlegungsschrift No. 2716707), compounds represented by the general formula (I) wherein $R_1$ and $R_2$ are a hydrogen, X is 2-amino-4-thiazolyl-2-syn-methoxyiminoacetyl group are mentioned but a practical embodiment with examples for preparing the compounds and antibacterial activities thereof are not described in this reference.

The present inventors have succeeded in preparing carbacephem compounds having various substituents at the 4-, 5- or 3-position. [numbering system is as shown in general formula (I)] These compounds are described in the specifications of Japanese Unexamined Published Patent Application No. 128591/79 (DE—A—2911786) and Japanese Patent Applications No. 162008/78.

Further, the present inventors have succeeded in preparing novel acylated carbacephems which are new antibiotics having strong antibacterial activities. These compounds are described in the specification of Japanese Patent Applications No. 34696/78, 122402/78, 127027/78, 133071/78, 162006/78, 162007/78, and 8409/79 (DE—A—2911787) and Japanese Patent Application No. 162008/78.

Among the compounds which were provided by the present inventors and represented by the general formula (II)

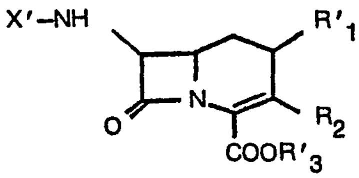

(II)

(wherein X′ is a conventional acyl group employed in the chemistry of cephalosporins and penicillins, $R'_1$ represents hydrogen atom, a lower alkyl group or lower acyloxy group $R'_3$ represents hydrogen atom or an ester-protecting group conventionally employed in the field of the chemistry of penicillins and cephalosporins, that is, an alkyl group having 1 to 5 carbon atoms such as methyl group, ethyl group, n-propyl group, isopropyl group, n-butyl group, isobutyl group or t-butyl group, a halogenated alkyl group having 1 to 5 carbon atoms such as chloromethyl group, 2,2,2-trichloroethyl group or 2,2,2-trifluoroethyl group, an arylmethyl group having 7 to 20 carbon atoms such as benzyl group, diphenylmethyl group, or triphenylmethyl group, an arylethyl group having 7 to 20 carbon atoms and having methoxy group or nitro group, on the phenyl ring, a substituted silyl group such as trimethylsilyl group or triphenylsilyl group or a group enzymatically or nonenzymatically readily eliminable *in vivo,* for example, a group represented by the general formula

$$-\underset{\displaystyle R_5}{\underset{|}{C}}HOCOR_4$$

wherein $R_5$ represents hydrogen atom or a lower alkyl group having 1 to 6 carbon atoms, a lower alkoxy group having 1 to 6 carbon atoms or phenyl group, etc. and $R_2$ has the same meaning as defined above, the acyl compounds represented by the formula (I)

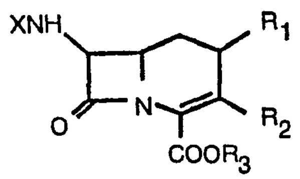

(I)

(wherein $R_1$, $R_2$, $R_3$ and X have the same meaning as defined above) which has a carbacephem ring represented by the formula (III) (mentioned below) are reported to have strong antimicrobial activity against Gram-positive and Gram-negative microorganisms in Japanese Patent Applications No. 122402/78, 133071/78, 162006/78, 162007/78 (DE—A—2911787). Especially the acyl compounds having the carbacephem ring represented by the general formula (I′)

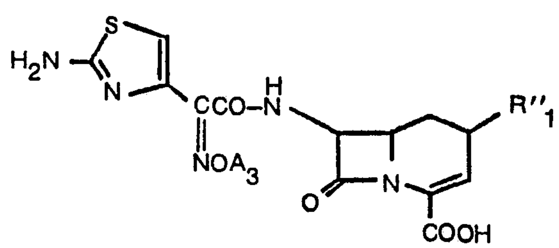

(I′)

wherein $A_3$ has the same significance as defied above, $R''_1$ represents a hydrogen or a methyl group and $OA_3$ has syn configuration, are reported to have strong antimicrobial activity against Gram-positive and Gram-negative microorganisms in the aforementioned patent applications. Hereinafter, compounds represented by the general formula (I), (II), (III), ..... are named Compound [I], Compound [II], Compound [III], ...., respectively.

However, the cephalosporin analogs mentioned above are prepared by totally synthetic methods using optically inactive starting compounds, and they are almost optically inactive dl-compounds except for a compound having a certain optically active acyl group for example D-phenylglycine, described in the specification of Japanese Patent Application No. 162006/78 (DE—A—2911787).

Accordingly, there is a demand for optically active analogs and methods for production thereof. To this end, it has now been found that certain optically active cephalosporin compounds can be prepared which have unexpectedly increased biological activity.

Summary of the Invention

In accordance with the present invention optically active active acyl compounds represented by the

formula (I) above are prepared from optically active compounds of the cephalosporin analogs represented by the general formula (III)

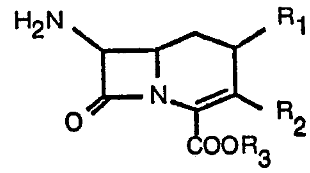

(III)

(wherein $R_1$, $R_2$ and $R_3$ have the same significance as defined above and hydrogens at the 6- and 7-positions have cis configuration). The compounds of the present invention have unexpectedly greater antibacterial activity, i.e. 2 to 4 times greater activity against various Gram-positive and Gram-negative microorganisms than the corresponding optically inactive dl-Compound [I].

Processes of producing optically active Compound [III] are described in detail in Reference Examples 9 to 11. The present applicant filed simultaneously an application relating to the optically active Compound [III] and processes of producing thereof (European Patent Application, publication No. 14475).

Further, additional to the known conventional acylating methods, the present inventors succeeded in preparing some of the desired compounds by optically selective acylation method, that is, the present inventors succeeded in preparing optically active cephalosporin analogs represented by the general formula (IV)

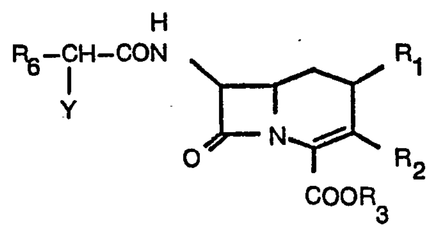

(IV)

(wherein $R_6$ represents a substituted or unsubstituted saturated or unsaturated six-membered carbocyclic or five-membered heterocyclic group wherein the substituent is selected from those specified for $A_1$ above, $R_1$, $R_2$ and $R_3$ have the same meanings defined above, Y represents a lower alkyl group having 1 to 5 carbon atoms, a hydroxy group, a carboxy group or an amino group, and the hydrogens at the 6- and 7-positions have cis configuration) by reacting optically inactive compound [III] or optically active compounds of the cephalosporin analogs represented by the following assumed absolute structural formula (III—1)

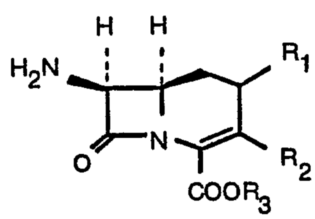

(III—1)

(wherein $R_1$, $R_2$, and $R_3$ have the same meaning as defined above) with $\alpha,\alpha$-disubstituted carboxylic acid represented by the formula (V)

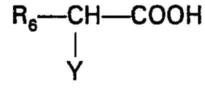

(V)

(wherein $R_6$ and Y have the same meaning as defined above or functionally equivalent reactive derivative in the presence of (1) a microorganism capable of producing optically active compounds of the cephalosporin analog represented by the general formula (IV), from the $\alpha,\alpha$-disubstituted carboxylic acid or reactive derivative thereof and the compound represented by the general formula (II) or (III—1) and belonging to the genus *Pseudomonas, Xanthomonas, Escherichia, Aeromonas, Achromobacter, Arthrobacter, Acetobacter, Alcaligenes, Kluyvera, Gluconobacter, Clostridium, Comamonas, Corynebacterium, Sarcina, Staphylococcus, Spirillum, Bacillus, Flavobacterium, Brevibacterium, Protaminobacter, Beneckea, Micrococcus, Proteus, Mycoplana* or *Rhodopseudomonas,* (2) a culture broth of the microorganism, (3) a treated matter of the culture broth or (4) an enzyme produced by the microorganism.

Optically inactive dl-compounds corresponding to Compound (II) are present as a mixture of equal amounts of optical isomers (III—1) and (III—2) which are mirror images of each other (enantiomers).

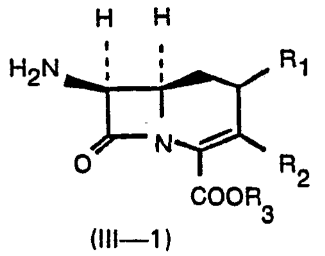

(III—1)

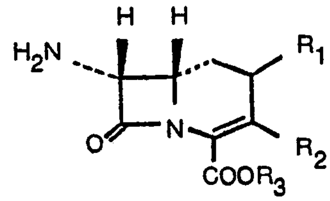

(III—2)

wherein $R_1$, $R_2$ and $R_3$ have the same significance as defined above.

The optically active compounds obtained by the processes described in Reference Examples below are assumed to have the absolute structure represented by the general formula (III—1) defined above on account of various properties, strong antimicrobial activity of the acyl compounds compared with the corresponding optically inactive dl-compound and the relationship between the absolute structure of cephalosporins and activities thereof.

In the following, the optically active compounds are described as having the absolute configuration of (6R, 7S), i.e. the configuration illustrated by the general formula (III—1) and in the following Examples and Reference Examples, the compounds are named according to the assumed absolute structural formula. It is needless to say that the optically active compounds are more useful as medicins and antimicrobial agents compared with optically inactive compounds and the compounds of the present invention are, therefore, useful as antibacterial agents which may be employed in manners well known in the art.

Detailed Description of the Invention

Optionally active cephalosporin analogs, according to the present invention, are represented by the general formula (I)

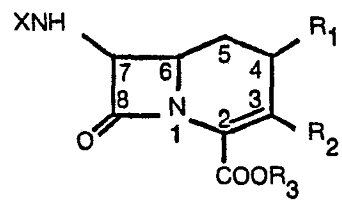

(I)

$R_1$ represents a hydrogen or a lower alkyl group having 1 to 5 carbon atoms such as methyl group, ethyl group, n-propyl group, i-propyl group, n-butyl group, i-butyl group, and t-butyl group, are exemplified. Particularly, methyl group is preferred. Further, it is preferable that the methyl group has the same configuration as the hydrogen atoms at the 6- and 7-positions, i.e. $4\alpha$-configuration in the structural formula (III—1). However, the compounds having $4\beta$-methyl group and the mixed compounds of $4\alpha$- and $4\beta$-methyl compounds are valuable enough.

As group $R_2$, a hydrogen or a halogen atom which is selected from chlorine, bromine or iodine atom is exemplified.

$R_3$ is a hydrogen or a protective group of carboxylic acid used in the chemistry of penicillins and cephalosporins.

As group $R_3$, the following are exemplified.

1. straight or branched alkyl group having 1 to 5 carbon atoms such as methyl group, ethyl group, n-propyl group, i-propyl group, n-butyl group, i-butyl group and t-butyl group.
2. straight or branched lower alkoxymethyl group having 1 to 5 carbon atoms such as methoxymethyl group and ethoxymethyl group.
3. straight or branched halogenated alkyl group having 1 to 5 carbon atoms such as chloromethyl group, 2,2,2-trichloromethyl group and 2,2,2-trifluoroethyl group.
4. lower alkylsulfonylethyl group such as methylsulfonylethyl group and ethylsulfonylethyl group.
5. arylmethyl group having 7 to 12 carbon atoms such as benzyl group, diphenylmethyl group, trityl group and triphenylmethyl group.
6. substituted silyl group such as trimethylsilyl group and triphenylsilyl group.
7. substituted arylmethyl group having 7 to 20 carbon atoms wherein the substituent is methoxy group, or nitro group and number of substituents on the phenyl ring is 1 to 5.

8. protective group of carboxylic acid represented by the general formula (VI)

$$-\underset{\displaystyle R_5}{\underset{|}{C}}HOCOR_4$$

wherein $R_4$ is a straight or branched lower alkyl group having 1 to 6 carbon atoms, a straight or branched lower alkoxy group having 1 to 6 carbon atoms, or a phenyl group, and $R_5$ is a hydrogen or a straight or branched lower alkyl group having 1 to 6 carbon atoms.

X represents an acyl group represented by the formula $X_1CO$ wherein $X_1$ represents the following two groups:

1) a group represented by the general formula

$$(A_1)_n B-\underset{\displaystyle A_2}{\underset{|}{C}}H-$$

[wherein B represents an unsaturated six membered carbocycle selected from cyclohexenyl group, cyclohexadienyl group and phenyl group or a five or six membered heterocycle such as furyl group, thienyl group, pyrrolyl group, thiazolyl group, oxazolyl group, isothiazolyl group, isoxazolyl group, imidazolyl group, pyrazolyl group, triazolyl group, tetrazolyl group, pyridinyl group, pyrimidinyl group, pyrazinyl group, pyridazinyl group, triazinyl group, and 5,6-dihydro-1,4-dithin-2-yl group, $A_1$ represents substituent(s) which is selected from hydroxyl group, a lower alkoxy group having 1 to 4 carbon atoms, a halo group, nitro group, amino group, aminomethyl group, and a methylsulfonamide group, n is a number from 0 to 5, and $A_2$ represents amino group, hydroxyl group, carboxyl group or sulfo group].

2) a group represented by the general formula

$$(A_1)_n B-\underset{\displaystyle NOA_3}{\underset{\|}{C}}-$$

[wherein $A_1$, B and n have the same significance as defined above and $A_3$ represents hydrogen atom, a lower alkyl group having 1 to 6 carbon atoms, a lower alkenyl group having 2 to 6 carbon atoms, a lower alkynyl group having 2 to 6 carbon atoms, a cycloalkyl group having 3 to 6 carbon atoms or an aryl group, those groups being unsubstituted or substituted with suitable substituent(s) which is selected from carboxyl group, cyano group, a halo group, carbamoyl group and a lower alkoxycarbonyl group having 1 to 4 carbon atoms].

In general, it is known that thiazolyl group represented by

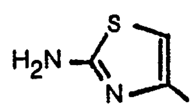

shows reversible interconversion with the thiazolinyl group, as shown below, and both are usually regarded as identical. In the present specification, both isomers are represented by thiazolyl group. Of course, Compound [I] includes the both isomers based on the reversible interconversion.

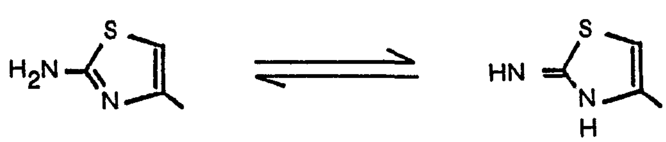

thiazolyl thiazolinyl

As for $OA_3$, syn configuration means isomer (A) in the following stereoisomers.

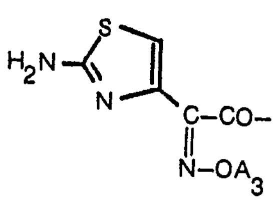

syn (A)

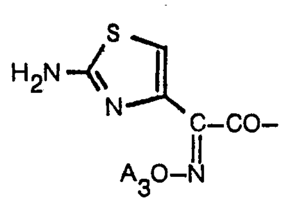

anti (B)

As the pharmaceutical acceptable salts, salts with inorganic or organic bases, for example, alkali metal salts such as sodium salts or potassium salts, alkaline earth metal salts such as magnesium salts, ammonium salts, trimethylamine salts, triethylamine salts, pyridine salts, procaine salts, purine salts, lysine salts, arginine salts and salts with inorganic or organic acid, for example, hydrochloride, sulfate, carbonate, phosphate, formate, trifluoroacetate, malate are exemplified. The pharmaceutically acceptable salts are prepared by the standard methods known in the art.

The compounds of the present invention are produced by reacting an optically active compounds represented by the general formula (III—1)

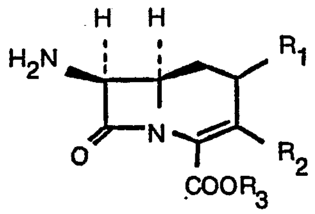

(III—1)

(wherein $R_1$, $R_2$ and $R_3$ have the same meaning as defined above) or a functionally equivalent compound with carboxylic acid represented by the general formula (VII)

$$X_2COOH \qquad (VII)$$

(wherein $X_2$ represents the following two groups:

1′) a group represented by the general formula:

$$(A'_1)_n B—\underset{\displaystyle A'_2}{\underset{|}{CH}}—$$

[wherein B and n have the same significance as fefined above, $A'_1$ represents a substituent which is selected from hydroxyl group, a protected hydroxyl group, a lower alkoxy group having 1 to 4 carbon atoms, a halo group, nitro group, a protected amino group, a protected aminomethyl group, and a methylsulfonamido group, and $A'_2$ represents, a protected amino group, hydroxyl group, a protected hydroxyl group, carboxyl group, a protected carboxyl group, sulfo group or a protected sulfo group].

2′) a group represented by the general formula:

$$(A'_1)_n B—\underset{\displaystyle NOA_4}{\underset{\|}{C}}—$$

[wherein $A'_1$, B and n have the same significance as defined above and $A_4$ represents hydrogen atom, a lower alkyl group having 1 to 6 carbon atoms, a lower alkenyl group having 2 to 6 carbon atoms, a lower alkynyl group having 2 to 6 carbon atoms, a cycloalkyl group having 3 to 6 carbon atoms or an aryl group, those groups being unsubstituted or substituted with a suitable substituent which is selected from a protected carboxyl group, cyano group, a halo group, carbamoyl group and a lower alkoxycarbonyl group having 1 to 4 carbon atoms], or with reactive derivatives of the carboxylic acid and, wherein, optionally deprotecting the protecting group in the group $X_2CO$— and/or —$COOR_3$ and optionally converting to pharmaceutically acceptable salts thereof. A concrete and known acylating method is described in Japanese Unexamined Patent Application No. 122402/53 (DE—A—2911787).

Some compounds of the present invention are produced by using optically inactive Compound [III]. In such case an optically inactive Compound [III] is reacted with carboxylic acid represented by the formula (VIII)

$$X_3COOH \qquad (VIII)$$

wherein $X_3$ represents the following group represented by the formula

$$(A'_1)_n B—\underset{\displaystyle A''_2}{\underset{|}{CH}}—$$

wherein $A'_1$, B and n have the same significance as defined above, $A''_2$ represents a protected amino group, hydroxyl group, a protected hydroxyl group, carboxyl group, a protected carboxyl group, sulfo group or protected sulfo group according to the similar method described above in case optically active Compound [III] is used as starting compound and the obtained diastereomeric mixture is separated by a conventional method.

Further, optically active cephalosporin analogs represented by the general formula (IV) are prepared by optically selective acylation between optically inactive Compound [III] or optically active Compound [III—1] and Compound [V] in the presence of (1) a microorganism capable of producing optically active compounds of the cephalosporin analog represented by the general formula (IV), from the $\alpha,\alpha$-disubstituted carboxylic acid or reactive derivative thereof and the compound represented by the general formula (III) or (III—1) and belonging to the genus *Pseudomonas, Xanthomonas, Escherichia, Aeromonas, Achromobacter, Arthrobacter, Acetobacter, Alcaligenes, Kluyvera, Gluconobacter, Clostridium, Comamonas, Corynebacterium, Sarcina, Straphylococcus, Spirillum, Bacillus, Flavobacterium, Brevibacterium, Protaminobacter, Beneckea, Micrococcus, Proteus, Mycoplana or Rhodopseudomonas,* (2) a culture broth of the microorganism, (3) a matter extracted from the culture broth or (4) an enzyme produced by the microorganism.

So far, enzymatic acylation of naturally occurring $\beta$-lactam compounds such as 6-aminopenicillanic acid which contains the penicillin ring system, 7-aminocephalosporanic acid and 7-aminodeacetoxycephalosporanic acid which contain the cephalosporin ring system has been widely reported. However no example of enzymatic acylation of a $\beta$-lactam compound synthetically prepared such as the present compound having carbacephem ring has been reported. The finding that such a microorganism has an ability of absolute optically selective acylation of a carbacephem compound synthetically prepared is quite new.

Optically inactive Compound [III] used in the present invention is produced according to the method described in Japanese Published Unexamined Patent Application No. 128591/79 (DE—A—2911786) and Japanese Patent Application No. 162008/78. A method of producing unpublished Compound [III] is described in detail in a Reference Example below. Compound [III—1] is produced according to the method described in Japanese Patent Application No. 14533/79 and 146488/79. A method of producing Compound [III—1] is described below.

As the reactive derivative of Compound [V], a compound which gives Compounds [V] by the hydrolysis with a microorganism used in the present invention, a culture broth of the microorganism, a treated matter of the culture broth and/or an enzyme produced by the microorganism, such as an alkylester with methyl group or ethyl group, a thioethylester with thioglycollic acid is mentioned.

The optically selective acylation reaction of Compound [III] with Compound [V] and the acylation reaction of Compound [III—1] with Compound [V] are carried out in the presence of an enzyme obtained from a microorganism capable of producing optically active Compound [I] or [I—1] by optically selective acylation of optically inactive Compound [III] or optical active Compound [III—1].

As the microorganism having an ability of such acylation reaction or optically selective acylation reaction such microorganism having an ability of producing optically active Compound [IV] or Compound [IV—1] from Compound [V] or a reactive derivative thereof and Compound [III] or Compound [III—1] and belonging to the genus *Aeromonas, Achromobacter, Arthrobacter, Acetobacter, Alcaligenes, Escherichia, Xanthomonas, Kluyvera, Gluconobacter, Clostridium, Comamonas, Corynebacterium, Sarcina, Staphylococus, Spirillum, Bacilus, Pseudomonas, Flavobacterium, Brevibacterium, Protaminobacter, Proteus, Beneckea, Micrococcus, Mycoplana* and *Rhodopseudomonas* are used. The following strains are examples of the microorganism.

| | |
|---|---|
| *Aeromonas hydrophila* | IFO 12634 |
| *Achromobacter aceris* | IFO 3320 |
| *Arthrobacter simplex* | ATCC 15799 |
| *Acetobacter aurantius* | IFO 3245 |
| *Acetobacter sp.* | ATCC 21760 |
| *Alcaligenes faecalis* | ATCC 8750 |
| *Escherichia coli* | ATCC 11105 |
| *Escherichia coli* | ATCC 13281 |
| *Xanthomonas citri* | IFO 3835 |
| *Xanthomonas physalidicola* | IFO 13555 |
| *Kluyvera citrophila* | ATCC 21285 |
| *Gluconobacter liquefaciens* | ATCC 14835 |
| *Gluconobacter dioxyacetonicus* | IFO 3271 |
| *Clostridium acetobutylicum* | IFO 3346 |
| *Comamonas terrigena* | IFO 12685 |
| *Corynebacterium tritici* | IFO 1216 |
| *Sarcina lutea* | ATCC 9341 |
| *Staphylococcus aureus* | IFO 3060 |
| *Spirillum metamorphum* | IFO 12012 |
| *Bacillus megaterium* | ATCC 14945 |
| *Pseudomonas melanogenum* | ATCC 17808 |
| *Pseudomonas aeruginosa* | IFO 3451 |
| *Flavobacterium sp.* | ATCC 21429 |
| *Brevibacterium cerinum* | ATCC 15112 |

| | |
|---|---|
| *Protaminobacter alboflavus* | IFO 13221 |
| *Proteus rettgeri* | ATCC 9250 |
| *Beneckea hyperoptica* | ATCC 15803 |
| *Micrococcus luteus* | AHU 1427 |
| *Mycoplana bullata* | IFO 13267 |
| *Rhodopseudomonas spheroides* | ATCC 21286 |

For carrying out the optically selective acylation reaction, the enzyme may be provided, more specifically, in any of the following forms:

1. As the culture liquor of the microorganism or treated matter thereof;
2. As cell bodies recovered from the culture broth by centrifugation which may be washed with saline water (usually about 1%), buffer solution and the like, or as a cell suspension;
3. As a disrupted cell suspension, i.e., a suspension of the cell bodies disrupted mechanically or chemically;
4. As a cell free extract, i.e., a liquid obtained by removing the disrupted cell bodies from the disrupted cell suspension; or
5. As a purified enzyme solution which is obtained by recovering the enzyme protein with ammonium sulfate from the cell free extract and subjecting the enzyme protein to gel filtration, ion-exchange cellulose column chromatography or ion-exchange sephadex® column chromatography (Pharmacia Fine Chemicals, Inc., Uppsala, Sweden).

Cells or the purified enzyme immobilized by a conventional method may be used.

The reaction is carried out at a temperature of 0 to 50°C, preferably 15 to 35°C and a pH of 5 to 8 in an inactive solvent which does not affect the reaction.

As the solvent, water is most preferably used. Organic solvents such as acetone, methanol, ethanol, N,N-dimethylformamide or dimethylsulfoxide may be used alone or in combination with water. It is effective to add phosphate buffer, Veronal buffer or citric acid buffer to control the pH in the reaction. Reaction time being influenced by the kind and concentration of enzymes, the kind and concentration of substrates, reaction temperature or reaction pH, is generally 30 minutes to 24 hours. It is most preferable to terminate the reaction when the reaction ratio reaches maximum.

The concentration of cells is preferably 1 to 50 mg by dry weight per 1 ml of the reaction solution. When a purified enzyme is used, it is appropriate to use the amount of the enzyme having the same activity as that of the dry cell. The substrate Compound [V] is used in an amount of 0.5 to 50 mg per 1 ml of the reaction solution. Compound [III] is used in an amount of 0.1 to 50 mg per 1 ml of the reaction solution.

When enzymes preventing the reaction such as β-lactamase or esterase are contained in the cell body, a mutant strain having a reduced productivity of the enzyme may be used. Further, inhibitors against such enzymes may be added in the reaction system to raise the reaction ratio.

After the completion of the reaction, isolation of the desired compound is carried out by a conventional method employed in the isolation and purification of organic compounds from culture liquors such as absorption using various carriers, ion-exchange chromatography, gel filtration or liquid-liquid extraction.

The invention includes within its scope pharmaceutical compositions comprising, as an active ingredient Compound [I] or a pharmaceutically acceptable salt thereof in association with a pharmaceutical carrier or diluent. The compounds of this invention are administered by parenteral (intramuscular, intraperitoneal, intravenous, or subcutaneous injection routes), oral or rectal route of administration and can be formulated in dosage forms appropriate for each route of administration.

Preparations according to this invention for parenteral administration includes sterile aqueous or non-aqueous solutions, suspensions, or emulsions. Examples of non-aqueous solvents or vehicles are propylene glycol, polyethylene glycol, vegetable oils, such as olive oil, and injectable organic esters such as ethyl oleate. Such dosage forms may also contain adjuvants such as preserving, wetting, emulsifying, and dispersing agents. They may be sterilized by, for example, filtration through a bacteria-retaining filter, by incorporating sterilizing agents into the compositions, by irradiating the compositions, or by heating the compositions. They can also be manufactured in the form of sterile solid compositions which can be dissolved in sterile water, or some other sterile injectable medium immediately before use.

Compositions for oral administration may be presented in a form suitable for absorption by the gastrointestinal tract. Tablets and capsules for oral administration may be in unit dose presentation form, and may contain conventional excipients such as binding agents, for examples, syrup, acacia, gelatin, sorbitol, tragacanth, or polyvinylpyrrolidone; fillers, for examples, lactose, sugar, maize-starch, calcium phosphate, sorbitol or glycine; lubricants, for example, magnesium stearate, talc, polyethylene glycol, silica; disintegrants, for example, potato starch or acceptable wetting agent such as sodium lauryl sulphate. The tablets may be coated according to methods well known in the arts. Oral liquid preparations may be in the form of aqueous or oily suspension, solution, emulsions, syrups, etc. or may be presented as a dry product, for reconstitution with water or other suitable vehicle before use. Such

liquid preparations may contain conventional additive such as suspending agents, for example, sorbitol syrup, methyl cellulose, glucose sugar syrup, gelatin, hydroxyethylcellulose, carboxymethylcellulose, aluminum stearate gel, emulsifying agents, for example, lecithin or sorbitan monooleate; non-aqueous vehicles, which may include edible oils, for example, almond oil, coconut oil, propylene glycol, or ethyl alcohol; preservatives, for example, methyl or propyl p-hydroxybenzoates or sorbic acid.

Compositions for rectal administration are preferably suppositories which may contain, in addition to the active substance, excipients such as cocoa butter or a suppository wax.

The dosage of active ingredient in the compositions of this invention may be varied; however, it is necessary that the amount of the active ingredient shall be such that a suitable dosage form is obtained. The selected dosage depends upon the desired therapeutic effect, on the route of administration, and on the duration of the treatment. Generally, dosage levels of between 5 to 350 mg/kg of body weight daily are administered to mammalian patients to achieve an antibiotic effect.

The present invention is explained in Examples below.

## Example 1

Preparation of (+) - cis - 7 - [2 - (2 - amino - 4 - thiazolyl) - 2 - syn - methoxyiminoacetamido] - 1 - azabicyclo[4,2,0]oct - 2 - en - 8 - on - 2 - carboxylic acid [the optically active compound of the composition represented by the general formula (I') wherein $A_3$ is $CH_3$, $R''_1$ is H, cis refers to the stereochemistry at the 6- and 7-positions and the same shall apply hereinafter].

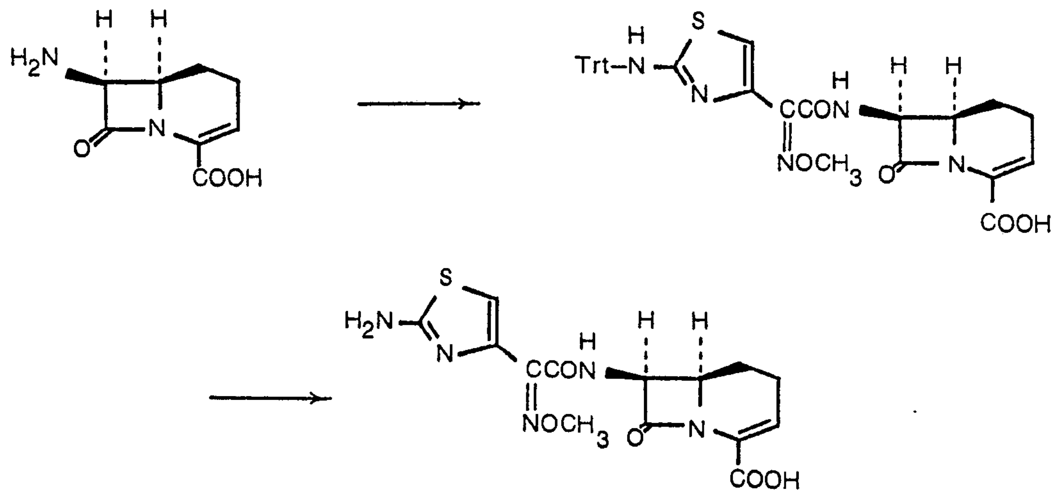

In this Example, 131.3 mg (0.30 m mole) of 2-(2-tritylamino-4-thiazolyl-2-syn-methoxyimino acetic acid is dissolved in 1 ml of anhydrous dichloromethane and to the solvent 4.1 μl of triethylamine is added at a temperature of −20°C.

After adding 61.7 mg of phosphorus pentachloride, the mixture is stirred at a temperature of −20°C for 30 minutes. The reaction mixture is concentrated under reduced pressure and the residue is dissolved in 1 ml of anhydrous tetrahydrofuran to obtain an acid chloride solution.

Separately, 40.2 mg (0.17 m mole) of the monohydrate of the hydrochloride of (+)-cis-7-amino-1-azabicyclo[4,2,0]oct-2-en-8-on-2-carboxylic acid obtained as in Reference Example 2 is dissolved in a mixture of 1 ml of tetrahydrofuran and 1 ml of water and 116.2 μl of triethyl amine is added thereto.

With stirring under ice cooling, the acid chloride solution prepared above is added dropwise to the solution and the mixture is allowed to react for one hour. The reaction solution is adusted to a pH of 2.0 with 5% hydrochloric acid and extracted three times with 10 ml of ethyl acetate. The ethyl acetate layer is washed with 10 ml of saturated saline solution, dried with sodium bicarbonate, and concentrated under reduced pressure to obtain 93 mg of a crude acyl compound {the optically active compound of represented by the general formula (I') wherein $A_3$ is $CH_3$, $R''_1$ is H and a hydrogen atom of $NH_2$ is replaced with Trt}. The compound is dissolved in 10 ml of 50% acetic acid and the solution is stirred at a temperature of 50°C for 30 minutes. After cooling to room temperature and removing deposited white precipitates by filtration, the reaction solution is concentrated and the residue is dissolved in a small amount of dimethylsulfoxide. The solution is charged on a column packed with 10 ml of HP—10. Elution is carried out with water to a mixture of water and methanol (1:2). Fractions showing an Rf value of 0.3 by silica gel thin layer chromatography [plate: Merck® Art. 5719 (Product of E. Merck & Co.], solvent:butanol:acetic acid:water = 4:1:1] are combined and concentrated under reduced pressure to obtain 13.5 mg (yield 22.4%) of white crystals of the desired compound.

Properties of the compound are as follows.

m.p. 172°C (decomposition)

$[\alpha]_D^{15°}$ +32.6° (DMSO, c=0.5)

IR (KBr) $\nu_{max}^{cm^{-1}}$: 1765, 1660, 1630, 1545,

PMR (DMSO-$d_6$)$\delta$: 9.26 (1H, d), 7.19 (2H, s), 6.75 (1H, s), 6.28 (1H, t), 5.50 (1H, d—d, J=8.9, 4.7), 3.83 (3H, s), 2.5—1.0 (4H, m)

These values coincide well with those of the corresponding dl-compound. From the strong antimicrobial activity, absolute configuration of this compound is assumed to be (6R, 7S).

Example 2

Preparation of (−) - cis - 7$\beta$ - [2 - (2 - amino - 4 - thiazolyl) - 2 - syn - methoxyimino - acetamido] - 4$\alpha$ - methyl - 1 - azabicyclo[4,2,0]oct - 2 - en - 8 - on - 2 - carboxylic acid [the optically active compound of the compound represented by the general formula (I') wherein $A_3$ and $R''_1$ are $CH_3$]

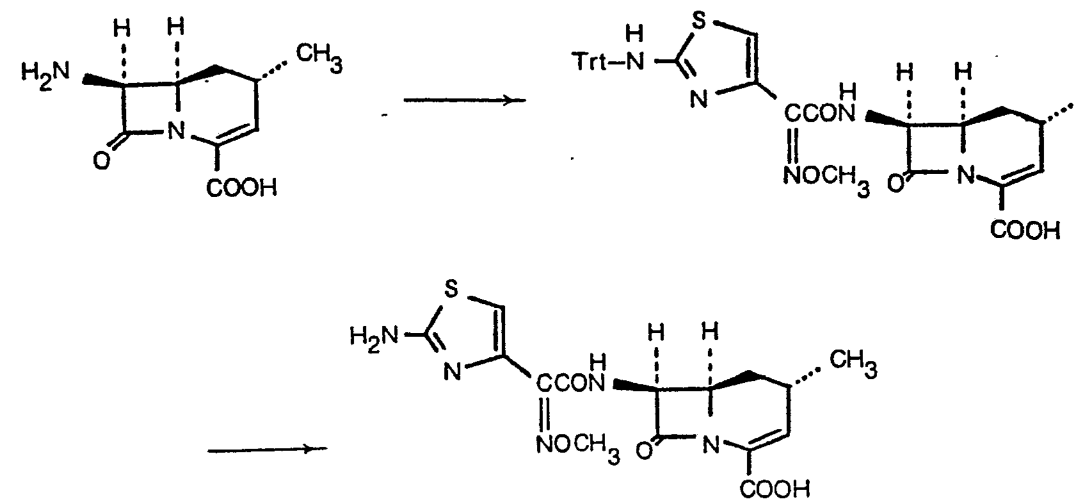

In this Example, 76 mg (0.17 m mole) of 2-(2-tritylamino-4-thiazolyl)-2-syn-methoxyimino acetic acid is dissolved in 1.52 ml of anhydrous dichloromethane and 17.3 mg (0.17 m mole) of triethylamine is added at a temperature of −15°C. After adding 35.7 mg (0.17 m mole) of phosphorus pentachloride, the mixture is stirred at a temperature of −15°C for 30 minutes. The reaction mixture is concentrated under reduced pressure and the residue is dissolved in 2 ml of anhydrous tetrahydrofuran to obtain an acid chloride solution.

Separately, 28 mg (0.10 m mole) of the dihydrate of the potassium salt of (−)-cis-7$\beta$-amino-4$\alpha$-methyl-1-azabicyclo[4,2,0]oct-2-en-8-on-2-carboxylic acid obtained as in Reference Example 4 is suspended in a mixture of 1.5 ml of tetrahydrofuran and water (1:1) and 36.3 mg (0.36 m mole) of triethylamine is added thereto to make a homogeneous solution. With stirring under ice cooling, the acid chloride solution is added dropwise to the solution and the mixture is allowed to react for 45 minutes. The reaction mixture is extracted 4 times with 3 ml of ethyl acetate and the ethyl acetate layer is washed with 5 ml of saturated saline solution. The washing is dried with sodium bicarbonate and concentrated under reduced pressure to obtain 107.1 mg of a crude acyl compound [the optically active compound of the compound represented by the general formula (I') wherein $A_3$ and $R''_1$ are $CH_3$ and a hydrogen atom of $NH_2$ is replaced with Trt]. The compound is dissolved in 4.5 ml of 50% acetic acid and the solution is stirred at a temperature of 50 to 55°C for 45 minutes. The solution is cooled to room temperature and the deposited white precipitate is removed by filtration. The cake is washed with 2 ml of 50% acetic acid. The filtrate and the washing are combined and concentrated under reduced pressure. The residue is dissolved in a small amount of dimethylsulfoxide and charged on a column packed with 10 ml of HP—10. Elution is carried out with a mixture of water and methanol (5:1 to 2:1). Fractions showing an Rf value of 0.54 by silica gel thin layer chromatography (the same condition hereinbefore is used) are combined and concentrated under reduced pressure to obtain 12.9 mg (yield 23.8%) of white crystals of the desired compound.

Properties of the compound are as follows.

m.p. about 180°C (decomposition)

$[\alpha]_D^{15°}$ −27° (DMSO, c=0.5)

IR (KBr) $\nu_{max}^{cm^{-1}}$: 1770, 1672, 1633, 1540

PMR (DMSO-$d_6$)$\delta$: 9.26 (1H, d, J=8.3), 7.18 (2H, s), 6.75 (1H, s), 6.31 (1H, d, J=5.1), 5.51 (1H, d—d, J=8.3, 5.0), 3.83 (3H, s), 1.67 (2H, m), 1.07 (3H, d, J=7.3)

These values coincide well with those of the corresponding dl-compound. From the strong antimicrobial activity, absolute configuration of this compound is assumed to be (4S, 6R, 7S).

Example 3

Preparation of (+) - cis - 7 - [(R) - 2 - phenyl - 2 - aminoacetamido] - 1 - azabicyclo-[4,2,0]oct - 2 - en - 8 - on - 2 - carboxylic acid [referred to as Compound (A) hereinafter]:

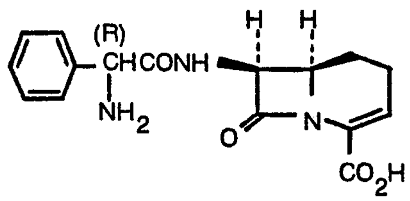

3—1) Preparation of disrupted cell suspension

a) Cultivation of a microorganism having an ability of optically selective acylation

As a seed strain, *Pseudomonas melanogenum* ATCC 17808 [Biological properties are described in Journal of the Agricultural Chemical Society of Japan, *37*, 71 (1963)] is used.

As a seed medium, an aqueous solution containing 1% polypeptone, 1% yeast extract, 0.5% meat extract, 0.5% sodium glutamate and 0.25% sodium chloride and adjusted at a pH of 7.0 with 5N—NaOH is used. One loopful seed strain is inoculated into 10 ml of the seed medium in a 50 ml tube and culturing is carried out at a temperature of 30°C for 24 hours. The whole amount of the seed medium is put into 300 ml of the culture medium in a 2 l Elrenmeyer flask and culturing is carried out with shaking at a temperature of 30°C. The composition of the culture medium is the same as that of the seed medium.

b) Preparation of cell suspension

After culturing for 24 hours, cell bodies are recovered from the culture broth by centrifugation and washed 2 times with 50 ml of 0.9% saline solution. The cells are suspended at a concentration of 40 mg/ml by dry weight in 1/30 M phosphate buffer.

The suspension (10 ml) is put in a 50 ml test tube and subjected to ultrasonic disintegration at 200 W for 2 minutes to prepare a disrupted cell suspension. In the treatment, a ultrasonic disintegrator Model UR 200P (product of Tomy Seiko Co., Ltd.) is used.

3—2) Preparation of substrate solution

120 mg of (±)-cis-7-amino-1-azabicyclo[4,2,0]oct-2-en-8-on-2-carboxylic acid obtained as in GO 2911786 and 500 mg of the hydrochloride of D-phenylglycinemethylester are added to 9 ml of 1/30 M potassium phosphate buffer (pH 6.5). 5N—KOH is added in small portions and the mixture is again adjusted to a pH of 6.5 to dissolve the two starting compounds. Finally, deionized water is added to make 10 ml of solution.

3—3) Enzyme reaction

In this step, 10 ml of the disrupted cell suspension is added to 10 ml of the substrate solution and enzyme reaction is carried out at a temperature of 30°C for 5 hours. The reaction is monitored by high speed liquid chromatography using TRI ROTAR® (product of Nippon Bunko Co., Ltd.) and Prepack column Nucleosil® 10C18 (product of Gaskuro Kogyo Co., Ltd.). Elution is carried out with 7% methanol-0.2 M $KH_2PO_4$ solution. Reaction reaches maximum in 5 hours.

3—4) Isolation and purification of the desired compound:

After the completion of reaction, cell bodies are removed from the reaction solution by centrifugation. The supernatant is concentrated under reduced pressure and charged on a column (1.6 cm width, 50 cm height) packed with 100 ml of Diaion® HP—10 (product of Mitsubishi Kasei Kogyo Co., Ltd.). After washing with 200 ml of deionized water, elution is carried out with 30% aqueous methanol solution. The eluate is collected in 10 ml fractions. Then, fraction Nos. 20 to 25 containing the desired compound are concentrated under reduced pressure to make 0.5 ml of a concentrate. The concentrate is charged on a column (1.6 cm width, 64.5 cm height) packed with 130 ml of Sephadex—LH20 (Pharmacia Fine Chemicals Inc.) and elution is carried out with a mixture of water and methanol (50:50 by volume, the same shall apply hereinafter). The eluate is collected in 5 ml fractions. The desired compound is eluted in fractions from 60 ml to 80 ml. The fractions are concentrated under reduced pressure to remove methanol and the residue is lyophilized to obtain 81 mg of a white powder.

Properties of the product are as follows.

$[\alpha]_D^{22c}$ ($H_2O$, c=0.5): +57.2°

IR (KBr) $\nu_{max}^{cm^{-1}}$: 1760, 1690, 1640

PMR ($D_2O$) $\delta$ (ppm): 7.51 (5H, s), 6.08 (1H, t, J=4.2Hz), 5.41 (1H, d, J=4.9Hz), 5.19 (1H, s), 3.83 (1H, octet, J=8.6, 3.7, 4.9Hz), 2.28—1.01 (4H, m)

The values mentioned above coincide with those of the less polar isomer in Example 6 of DE—A—2911787. Based on the data above, the white powder is identified as the desired compound.

From the strong antimicrobial activity, absolute configuration of this compound is assumed to be (6R, 7S).

Example 4

Preparation of (+) - cis - 7β - [(R) - 2 - phenyl - 2 - aminoacetamido] - 4α - methyl - 1 - azabicyclo[4,2,0]oct - 2 - en - 8 - on - 2 - carboxylic acid [referred to as Compound (B) hereinafter]:

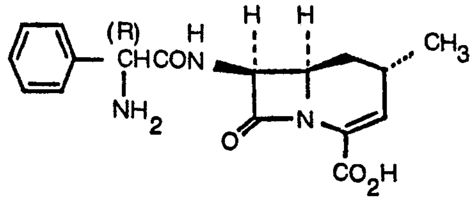

4—1) Preparation of cell suspension

A cell suspension is prepared as in Example 3—1) except that as the seed strain *Xanthomonas citri* IFO 3825 [Biological properties are described in Bergey's Manual of Determinative Bacteriology VI, P.156 (1948)] is used.

4—2) Preparation of substrate solution

200 mg of the trifluoroacetate of (±)-cis-7β-amino-4α-methyl-1-azabicyclo[4,2,0]oct-2-en-8-on-2-carboxylic acid obtained as in DE—A—2911786 and 500 mg of the hydrochloride of D-phenylglycinemethylester are added to 9 ml of 1/30 M potassium phosphate buffer (pH 6.5). 5N—KOH is added in small portions and the mixture is again adjusted to a pH of 6.5 to dissolve the two starting compounds. Finally, deionized water is added to make 10 ml of solution.

4—3) Enzyme reaction and 4—4) Isolation and Purification of the desired compound.

In this step, 10 ml of the disrupted cell suspension is added to 10 ml of the substrate solution and enzyme reaction is carried out at a temperature of 30°C for 4 hours. The reaction is monitored as in Example 3. Reaction reaches maximum in 4 hours. Then, the same procedure as in Example 3—4) is repeated to obtain 76 mg of a white powder.

Properties of the product are as follows.

$[\alpha]_D^{15°}$ ($H_2O$, c=0.52): +4,23°

IR (KBr) $\nu_{max}^{cm^{-1}}$: 3420, 1760, 1695, 1633

PMR ($D_2O$) δ (ppm): 7.51 (5H, s), 6.10 (1H, d, J=5.1Hz), 5.47 (1H, d, J=4.7Hz), 5.19 (1H, s), 3.89 (1H, m), 2.45 (1H, m), 1.44—1.04 (2H, m), 1.00 (3H, d, J=7.4Hz)

The values mentioned above coincide with those of the less polar isomer obtained in Example 12 of DE—A—2911787. Based on the data, the white powder is identified as the desired compound. From the strong antimicrobial activity, absolute configuration of this compound is assumed to be (4S, 6R, 7S).

Example 5

Preparation of Compound (A) (Alternative method). As the seed strain, *Escherichia coli* ATCC 11105 [Biological proeprties are described in Bergey's Manual of Determinative Bacteriology VIII P.195 (1974)]. The same procedure as in Example 3 is repeated except that enzyme reaction is carried out for 12 hours to obtain 30 mg of a white powder. Properties of the product coincide well with those of the compound obtained in Example 3.

Example 6

Preparation of (+) - cis - 7 - [(R) - 2 - p - hydroxyphenyl - 2 - aminoacetamido] - 1 - azabicyclo[4,2,0]oct - 2 - en - 8 - on - 2 - carboxylic acid

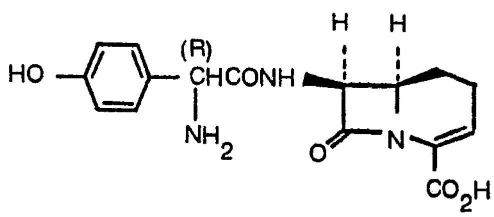

6—1) Preparation of cell suspension

a) Cultivation of a microorganism having an ability of optically selective acylation.

The same procedure as in Example 3—1) a) is repeated except that as the seed strain, *Kluyuera citrophila* ATCC 21285 [Biological properties are described in J. General Applied Microbiology *3*, 28—31 (1957)] is used.

b) Preparation of cell suspension

After culturing for 24 hours, cell bodies are recovered from the culture broth by centrifugation and washed 2 times with 50 ml of 0.9% saline solution. The cells are suspended at a concentration of 40 mg/ml by dry weight in 1/30 M phosphate buffer.

6—2) Preparation of substrate solution

120 mg of (±)-cis-7-amino-1-azabicyclo[4,2,0]oct-2-en-8-on-2-carboxylic acid obtained as in DE—A—2911786 and 500 mg of the hydrochloride of D-p-hydroxyphenylglycinemethylester are added in 9 ml of 1/30 M potassium phosphate buffer (pH 6.5). 5N—KOH is added in small portions and the mixture is again adjusted to a pH of 6.5 to dissolve the two starting compounds. Finally, deionized water is added to make 10 ml of solution.

6—3) Enzyme reaction and 6—4) Isolation and Purification of the desired compound

In this step, 10 ml of the cell suspension is added to 10 ml of the substrate solution and an enzyme reaction is carried out at a temperature of 30°C for 20 hours. The reaction is monitored as in Example 3. Reaction reaches maximum in 20 hours.

The same procedure as in Example 3—4) is repeated to obtain 65 mg of a white powder. Properties of the product are as follows.

$[\alpha]_D^{20^\circ} = +107.5°$ [c=0.5, 1 M phosphate bufer (pH 7.0)]

IR (KBr) $\nu_{max}^{cm^{-1}}$: 3450, 3290, 3090, 1760, 1700 (sh), 1685

PMR ($D_2O$) $\delta$(ppm): 7.36 (2H, d, J=8.8Hz), 6.95 (2H, d, J=8.8Hz), 6.06 (1H, t, J=3.9Hz), 5.40 (1H, d, J=4.6Hz), 5.12 (1H, s), 3.84 (1H, m), 2.22 (2H, m), 1.62 (1H, m), 1.12 (1H, m)

Based on the data mentioned above, the white powder is identified as the desired compound. From the strong antimicrobial activity, absolute configuration of this compound is assumed to be (6R, 7S).

Example 7

Preparation of (+) - cis - 7β - [(R) - 2 - p - hydroxyphenyl - 2 - aminoacetamido] - 4α - methyl - 1 - azabicyclo[4,2,0]oct - 2 - en - 8 - on - 2 - carboxylic acid.

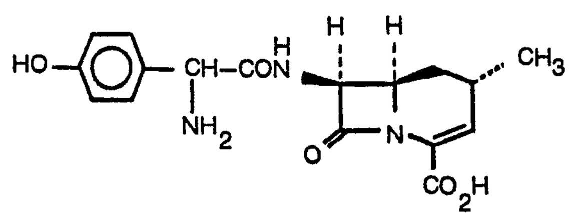

7—1) Preparation of cell suspension

A cell suspension is prepared as in Example 6 except that as the seed strain *Xanthomonas citri* IFO 3835 is used.

7—2) Preparation of substrate solution

A substrate solution is prepared as in Example 4—2).

7—3) Enzyme reaction and 7—4) Isolation and Purification of the desired compound:

The same procedures as in Example 3—3) and 3—4) are repeated using the cell suspension and the substrate solution prepared above to obtain 75 mg of a white powder. The reaction is carried out for 3 hours. Properties of the product are as follows.

$[\alpha]_D^{20^\circ} = +12.8°$ [c=0.5, 1 M phosphate buffer (pH 7.0)]

IR (KBr) $\nu_{max}^{cm^{-1}}$: 3420, 3260, 1760, 1685

PMR ($D_2O$) $\delta$(ppm): 7.35 (2H, d, J=8.0Hz), 6.96 (2H, d, J=8.0Hz), 6.10 (1H, d, J=5.1Hz), 5.45 (1H, d, J=4.9Hz), 5.11 (1H, s), 3.92 (1H, m), 2.45 (1H, m), 1.50—1.08 (2H, m), 1.01 (3H, d, J=7.1Hz)

Based on the data mentioned above, the white powder is identified as the desired compound. From the strong antimicrobial activity, absolute configuration of this compound is assumed to be (4S, 6R, 7S).

Example 8

Preparation of Compound (A) (Alternative method). The same procedure as in Example 3 is repeated except that 120 mg of (+)-cis-7-amino-1-azabicyclo[4,2,0]oct-2-en-8-on-2-carboxylic acid obtained in Reference Example 2 is used in place of (±)-cis-7-amino-1-azabicyclo[4,2,0]oct-2-en-8-on-2-carboxylic acid to obtain 160 mg of a white powder. Properties of the product coincide with those of the compound obtained as in Example 3.

Example 9

Preparation of Compound (B) (Alternative method). The same procedure as in Example 4 is repeated except that 150 mg of (—)-cis-7β-amino-4α-methyl-1-azabicyclo[4,2,0]oct-2-en-8-on-2-carboxylic acid obtained in Reference Example 4 is used in place of the trifluoroacetate (±)-cis-7β-amino-4α-methyl-1-azabicyclo[4,2,0]oct-2-en-8-on-2-carboxylic acid to obtain 150 mg of a white powder. Properties of the product coincide with those of the compound obtained in Example 4.

Example 10

Antibacterial activities of the compounds obtained in Examples 3, 4, 6 and 7 are illustrated in Table 1. The compounds obtained in Reference Example below and cefazolin are used as controls. Heart Infusion Agar Dilution Method (pH 7.2) is used.

In Table 1, the numbers of the compounds represent the following compounds and the letters A to O represent the following strains.

1. (+) compound obtained in Example 3.
2. (±) compound obtained in Example 6 of GO 2911787.
3. (—) compound obtained in Example 6 of GO 2911787.
4. (+) compound obtained in Example 4.
5. (±) compound obtained in Example 12 of GO 2911787.
6. (—) compound obtained in Example 12 of GO 2911787.
7. (+) compound obtained in Example 6.
8. (+) compound obtained in Example 47 of GO 2911787.
9. (+) compound obtained in Example 7.
10. (+) compound obtained in Example 44 of GO 2911787.
11. Cefazolin (control).

MIC of the (+) compounds obtained in Examples 6 and 12 of GO 2911787 coincide with MIC of the (+) compounds obtained in Examples 3 and 4.

A. *Staphylococcus aureus* 209—P
B. *Staphylococcus aureus* Smith
C. *Staphylococcus epidermidis*
D. *Escherichia coli* NIHJC—2
E. *Escherichia coli* Juhl
F. *Klebsiella pneumoniae* 8045
G. *Klebsiella pneumoniae* Y—60
H. *Serratia marcescens* T—26
I. *Serratia marcescens* T—55
J. *Proteus mirabilis* 1287
K. *Proteus vulgaris* 6897
L. *Proteus morganii* KY 4298
M. *Proteus rettgeri* KY 4289
N. *Pseudomonas aeruginosa* 145
O. *Pseudomonas putida* F264

TABLE 1

| Compound / Strain | 1 | 2 | 3 | 4 | 5 | 6 | 7 | 8 | 9 | 10 | 11 |
|---|---|---|---|---|---|---|---|---|---|---|---|
| A | 0.4 | 0.4 | – | 0.78 | 0.78 | – | 0.4 | 1.56 | 0.4 | 0.4 | <0.05 |
| B | 6.25 | 12.5 | – | 6.25 | 6.25 | – | 3.12 | 12.5 | 1.56 | 1.56 | 0.4 |
| C | 3.12 | 6.25 | – | 3.12 | 6.25 | – | 1.56 | 6.25 | 0.78 | 1.56 | 0.78 |
| D | 3.12 | 12.5 | 50 | 12.5 | 12.5 | 50 | 3.12 | 12.5 | 6.25 | 6.25 | 1.56 |
| E | 3.12 | 12.5 | 100 | 12.5 | 12.5 | 100 | 6.25 | 12.5 | 6.25 | 6.25 | 1.56 |
| F | 0.78 | 3.12 | 50 | 0.78 | 3.12 | 50 | 0.78 | 3.12 | 3.12 | 12.5 | 0.78 |
| G | 3.12 | 12.5 | 50 | 25 | >100 | 50 | 3.12 | 12.5 | 100 | >100 | 3.12 |
| H | – | >100 | – | >50 | >100 | – | 100 | – | 50 | 100 | >100 |
| I | 12.5 | 25 | 100 | 25 | >100 | 100 | 6.25 | 50 | 12.5 | 50 | 50 |
| J | 12.5 | 25 | 25 | 25 | 50 | 25 | 50 | 50 | 25 | 50 | 12.5 |
| K | 50 | 25 | 25 | 25 | 50 | 25 | 100 | – | 100 | >100 | 12.5 |
| L | 50 | 100 | 50 | 25 | 100 | 50 | 100 | – | 100 | >100 | >100 |
| M | 12.5 | 50 | 6.25 | 12.5 | >100 | – | 12.5 | 25 | – | – | 25 |
| N | – | >100 | – | >50 | >100 | – | >100 | – | – | – | >100 |
| O | 100 | >100 | – | >50 | 100 | – | 100 | – | 50 | 100 | >100 |

Example 11

Preparation of t-butyl ester of (±) - cis - 7 - (2 - phenyl - 2 - t - butyloxycarbonyl - aminoacetamido) - 3 - chloro - 1 - azabicyclo[4,2,0]oct - 2 - en - 8 - on - 2 - carboxylic acid

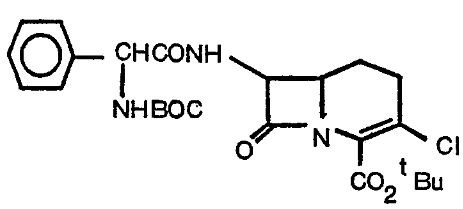

In this example, 150 mg (0.55 mmole) of (±)-cis-7-amino-3-chloro-2-t-butyloxycarbonyl-1-azabicyclo[4,2,0]oct-2-en-8-one is dissolved in 3 ml of anhydrous methylene chloride. 166 mg (0.66 mmole) of (R)-N-t-butyloxycarbonylphenylglycine is dissolved in 5 ml of anhydrous tetrahydrofuran and the solution is cooled to −15 to −10°C. 0.66 ml (0.66 mmole) of 1N-N-methylmorpholine-tetrahydrofuran and 0.66 ml (0.66 mmole) of 1N-$^{i}$Bu chloroformate-tetrahydrofuran are added dropwise and the mixture is stirred at the same temperature for 20 minutes. The above-prepared amine solution is added dropwise to the mixture while maintaining the same temperature and temperature of the mixture is raised to room temperature gradually. The mixture is stirred at room temperature overnight. 10 ml of methylene chloride is added to the reaction mixture and the mixture is washed with 10% citric acid, saturated aqueous sodium bicarbonate and saturated aqueous sodium chloride in this order, and dried with anhydrous sodium sulfate. The mixture is concentrated under reduced pressure and purified by silica gel chromatography (silica gel; 20 g of C—200 produced by Wako Junyaku Co., Ltd., Japan, solvent; ethyl acetate:n-hexane = 1:5 by volume, this is same hereinafter) to obtain 124 mg (yield 44.6%) of the desired compound (mixture of the diasteroisomers) having the following properties.

IR (KBr) $\nu_{max}^{cm^{-1}}$: 1780, 1730, 1680, 1655, 1550

NMR ($CDCl_3$) $\delta$ (ppm): 7.34 (5/2H, s), 7.31 (5/2H, s), 6.93 (1H, m), 5.63 (1H, m), 5.30 (1/2H, dd, J=5.4, 6.8Hz), 5.11—5.22 (3/2H, m), 3.76—3.91 (1H, m), 2.33—2.66 (2H, m), 1.52 (9H, s), 1.41 (9H, s), 0.92—1.97 (2H, m)

Example 12

Preparation of (6R,7S) 7 - (R) - phenylglycinamido - 3 - chloro - 1 - azabicyclo[4,2,0]oct - 2 - en - 8 - on - 2 - carboxylic acid (C) and (6S, 7R) 7 - (R) - phenylglycinamido - 3 - chloro - 1 - azabicyclo[4,2,0]oct - 2 - en - 8 - on - 2 - carboxylic acid (D)

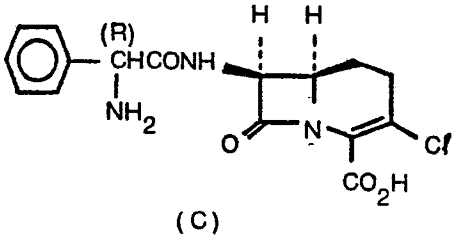

(C)

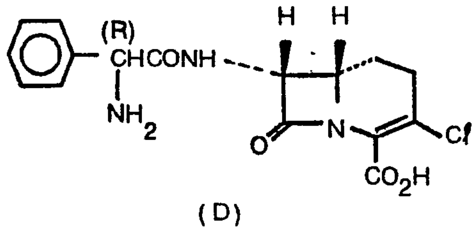

(D)

In this example, 1 ml of methylene chloride and 1 ml of trifluoroacetic acid are added to 128.4 mg (0.25 mmole) of t-butyl ester of (±) - cis - 7 - (2 - phenyl - 2 - t - butyloxycarbonylamino-acetamido) - 3 - chloro - 1 - azabicyclo[4,2,0]oct - 2 - en - 8 - on - 2 - carboxylic acid under ice cooling and the mixture is stirred at the same temperature for 1.5 hours. The solvent is distilled off and the resultant oily material is subjected to high speed liquid chromatography [column: Microbondapak® C—18 (product of Waters Co.), solvent: 7% methanol and 0.2 N $KH_2PO_4$] to separate diastereo-isomers. Each separated eluate fraction is concentrated under reduced pressure and desalted using 10 ml of Diaion HP—10 resin (solvent; methanol:water = 1:1) to obtain 9.4 mg of more polar isomer (D) and 7.6 mg of less polar isomer (C) (total yield 19.1%).

More polar isomer (D)

$[\alpha]_D^{21°} = -75.8°$ [c=0.4, $H_2O$]

Melting point: 300°C or more (browning)

IR (KBr) $\nu_{max}^{cm^{-1}}$: 1765, 1700, 1550

NMR ($D_2O$) $\delta$ (ppm): 7.49 (5H, s), 5.16 (1H, d, J=4.7Hz), 5.05 (1H, s), 3.78—4.03 (1H, m), 2.53—2.67 (2H, m), 1.26—2.09 (2H, m)

Less polar isomer (C)

$[\alpha]_D^{21°} = +34.0°$ [c=0.35, $H_2O$]

Melting point: 300°C or more (browning)

IR (KBr) $\nu_{max}^{cm^{-1}}$: 1770, 1700, 1620

NMR ($D_2O$) $\delta$ (ppm): 7.51 (5H, s), 5.36 (1H, d, J=4.6Hz), 5.19 (1H, s), 3.83—4.00 (1H, m), 2.41—2.56 (2H, m), 1.49—1.76 (1H, m), 1.14—1.45 (1H, m)

As described below, the less polar isomer (C) exhibits a greater antimicrobial activity and is assumed as the (6R, 7S) absolute configuration.

Example 13

Preparation of (±) - cis - 7 - phenylacetamido - 3 - chloro - 1 - azabicyclo[4,2,0]oct - 2 - en - 8 - on - 2 - carboxylic acid:

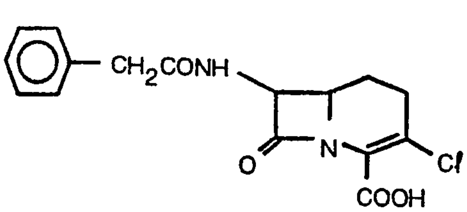

In this example, 150 mg (0.45 mmole) of trifluoroacetate of (±)-cis-7-amino-3-chloro-1-azabicyclo[4,2,0]oct-2-en-8-on-2-carboxylic acid is suspensed in a mixed solvent of 2 ml of water and 2 ml of acetone, 134 mg (1.5 mmole) of sodium bicarbonate is added thereto to make a homogeneous solution. To the solution is added dropwise a solution of 84.2 mg (0.54 mole) of phenylacetyl chloride in 0.5 ml of acetone under ice cooling in one hour. The mixture is stirred for 3 hours, adjusted to pH 2 with 1 N hydrochloric acid and extracted 5 times with each 2 ml of ethyl acetate. The extract is concentrated under reduced pressure and the residue is dried to obtain 80 mg (55.0%) of the desired compound.

IR (KBr) $\nu_{max}^{cm^{-1}}$: 1790, 1705, 1630, 1560

NMR ($CD_3OD$) $\delta$ (ppm): 7.29 (5H, s), 5.36 (1H, d, J=5Hz), 3.79—3.99 (1H, m), 2.56—2.75 (2H, m), 1.17—2.02 (2H, m)

Example 14

Preparation of (6R, 7S) 7 - (R) - p - hydroxyphenylglycinamido - 3 - chloro - 1 - azabicyclo-[4,2,0]oct - 2 - en - 8 - on - 2 - carboxylic acid:

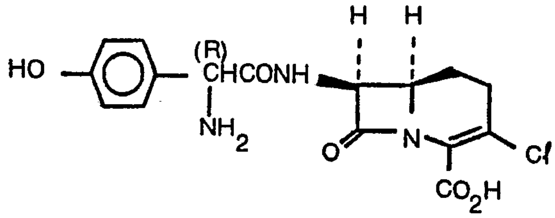

In this example, 1 ml of methylene chloride and 1 ml of trifluoroacetic acid are added under ice cooling to 104.3 mg (20 mmole) of t-butyl ester of (±) - cis - 7 - [2 - (p - hydroxyphenyl) - 2 - t - butyloxycarbonylaminoacetamido] - 3 - chloro - 1 - azabicyclo[4,2,0]oct - 2 - en - 8 - on - 2 - carboxylic acid (mixture of the diastereoisomers) synthesized from t-butyl ester of (±) - cis - 7 - amino - 3 - chloro - 1 - azabicyclo[4,2,0]oct - 2 - en - 8 - on - 2 - carboxylic acid and N - t - butyloxycarbonyl - (R) - p - hydroxyphenylglycine. The mixture is stirred at the temperature for 1.5 hours and concentrated under reduced pressure. The resultant oily material is subjected to high speed liquid chromatography [column: Microbondapak C—18, solvent: 7%-methanol-0.2 N potassium dihydrogen phosphate) to separate the diastereoisomers. Fractions containing the less polar isomer are concentrated under reduced pressure and desalted by 10 ml of HP—10 resin (solvent, methanol:water = 1:1) to obtain 10.5 mg (28.7%) of the desired compound.

$[\alpha]_D^{20°}$: +44° (c=0.25, 1 M phosphate buffer, pH 7.0)

IR (KBr) $\nu_{max}^{cm^{-1}}$: 1765, 1695, 1615, 1520

NMR ($D_2O$) $\delta$ (ppm): 7.36 (2H, d, J=8.8Hz), 6.96 (2H, d, J=8.8Hz), 5.36 (1H, d, J=4.6Hz), 5.11 (1H, s), 3.81—4.00 (1H, m), 2.42—2.58 (2H, m), 1.59—1.77 (1H, m), 1.17—1.48 (1H, m)

Antibacterial activity of the compounds obtained in Examples 12 and 14 is shown in the following table. The activity is determined by the Heart Infusion Agar Dilution Method (pH 7.2). Cephalexin and cefazolin are used as reference. From the antimicrobial activity, absolute configuration of this compound is assumed to be (6R, 7S).

TABLE 2

| Microorganism | MIC (μg/ml) | | | |
|---|---|---|---|---|
| | a | b | c | d |
| Staphylococcus aureus 209–p | 0.1 | 0.4 | 0.2 | ≤0.05 |
| Staphylococcus aureus Smith | 1.56 | 1.56 | 3.12 | 0.4 |
| Staphylococcus epidermidis | 1.56 | 1.56 | 3.12 | 0.78 |
| Escherichia coli NIHJC–2 | 1.56 | 1.56 | 12.5 | 1.56 |
| Escherichia coli Juhl | 1.56 | 1.56 | 12.5 | 1.56 |
| Klebsiella pneumoniae 8045 | 0.2 | 0.78 | 3.12 | 0.78 |
| Klebsiella pneumoniae Y–60 | 6.25 | 12.5 | 50 | 3.12 |
| Serratia mercescens T–55 | 6.25 | 6.25 | 50 | 50 |
| Proteus mirabilis 1287 | 3.12 | 3.12 | 25 | 12.5 |
| Proteus vulgaris 6897 | 100 | 100 | 25 | 12.5 |
| Proteus morganii KY4298 | >100 | >100 | >100 | >100 |
| Pseudomonas putida F264 | 100 | 100 | >100 | >100 |
| Proteus rettgeri KY4289 | 100 | 100 | >100 | 25 |

a: The compound (C) obtained in Example 12.

b: The compound obtained in Example 14.

c: Cephalexin.

d: Cefazolin.

## Example 15

Preparation of Compound (A) (Alternative method):

15—1) Cultivation of microorganisms

As the seed strain, the following strains are used.

*Aeromonas hydrophila* IFO 12634
*Achromobacter aceris* IFO 3320
*Arthrobacter simplex* ATCC 15799
*Acetobacter aurantius* IFO 3245
*Acetobacter sp.* ATCC 21760
*Alcaligenes faecalis* ATCC 8750
*Escherichia coli* ATCC 13281
*Xanthomonas physalidicola* IFO 13555
*Gluconobacter liquefaciens* ATCC 14835
*Gluconobacter dioxyacetonicus* IFO 3271
*Comamonas terrigena* IFO 12685
*Corynebacterium tritici* IFO 1216
*Sarcina lutea* ATCC 9341
*Staphylococcus aureus* IFO 3060
*Spirillum methamorphum* IFO 12012
*Bacillus megaterium* ATCC 14945
*Pseudomonas aeruginosa* IFO 3451

*Flavobacterium sp.* ATCC 21429
*Brevibacterium cerinum* ATCC 15112
*Protaminobacter alboflavus* IFO 13221
*Proteus rettgeri* ATCC 9250
*Beneckea hyperoptica* ATCC 15802
*Micrococcus luteus* AHU 1427
*Mycoplana bullata* IFO 13267
*Mycoplana dimorpha* IFO 13213
*Rhodopseudomonas spheroides* ATCC 21286

As a medium, an aqueous solution containing 1% meat extract, 1% peptone, 0.3% sodium chloride, 0.5% yeast extract and adjusted at a pH of 7.2 with 5N—NaOH is used. One loopful seed strain is inoculated into a 300 ml Erlenmeyer flask containing 30 ml of the medium and culturing is carried out with shaking at a temperature of 30°C for 24 hours. Cell bodies are recovered from the culture broth by filtration and washed with 5 ml of 0.9% sodium chloride solution. The cells are again recovered by centrifugation and suspended at a concentration of 40 mg/ml by dry weight in 1/30 M phosphate buffer (pH 6.5).

15—2) Preparation of substrate solution
A substrate solution is obtained as in Example 3—2). That is, 123 mg of (±)-cis-7-amino-1-azabicyclo[4,2,0]oct-2-en-8-on-2-carboxylic acid and 400 mg of the hydrochloride of D-phenylglycinemethylester are added in 15 ml of 1/30 M phosphate buffer (pH 6.5). 5N—KOH is added in small portions and the mixture is adjusted to a pH of 6.5 to dissolve the two starting compounds. Finally, deionized water is added to make 20 ml of solution.

15—3) Enzyme reaction
The same procedure as in Example 6 is carried out using 0.5 ml of the cell suspension obtained in 15—1) and 0.5 ml of the substrate solution. The reaction is carried out at a temperature of 30°C for 24 hours.

15—4) Identification of the desired compound
Reaction is monitored by high speed liquid chromatography in the same manner as in Example 1.
As apparent from Table 3, the desired more polar diastereoisomer, i.e. (—) - cis - 7 - [(R) - 2 - phenyl - 2 - aminoacetamido] - 1 - azabicyclo[4,2,0]oct - 2 - en - 8 - on - 2 - carboxylic acid [DE—A—2911787] is not formed at all and only the desired less polar isomer, i.e. (+) - cis - 7 - [(R) - 2 - phenyl - 2 - aminoacetamido] - 1 - azabicyclo[4,2,0]oct - 2 - en - 8 - on - 2 - carboxylic acid is formed. The enzymes produced by all strains in Table 3 have an ability to effect optically selective acylation of cephalosporin analogs.

TABLE 3

| Microorganism | The amount of * Compound (A) produced (mg) |
|---|---|
| *Aeromonas hydrophila* IFO 12634 | 0.05 |
| *Achromobacter aceris* IFO 3320 | 0.08 |
| *Arthrobacter simplex* ATCC 15799 | 0.06 |
| *Acetobacter aurantius* IFO 3245 | 0.04 |
| *Acetobacter sp.* ATCC 21760 | 1.02 |
| *Alcaligenes faecalis* ATCC 8750 | 0.04 |
| *Escherichia coli* ATCC 13281 | 0.25 |
| *Xanthomonas physalidicola* IFO 13555 | 0.95 |
| *Gluconobacter liquefaciens* ATCC 14835 | 0.13 |
| *Gluconobacter dioxyacetonicus* IFO 3271 | 0.20 |
| *Comamonas terrigena* IFO 12685 | 0.03 |
| *Corynebacterium tritici* IFO 1216 | 0.07 |
| *Sarcina lutea* ATCC 9341 | 0.05 |
| *Staphylococcus aureus* IFO 3060 | 0.09 |
| *Spirillum methamorphum* IFO 12012 | 0.18 |
| *Bacillus megaterium* ATCC 14945 | 0.05 |
| *Pseudomonas aeruginosa* IFO 3451 | 0.03 |
| *Flavobacterium sp.* ATCC 21429 | 0.04 |
| *Brevibacterium cerinum* ATCC 15112 | 0.20 |
| *Protaminobacter alboflavus* IFO 13221 | 1.21 |
| *Proteus rettgeri* ATCC 9250 | 0.09 |
| *Beneckea hyperoptica* ATCC 15803 | 0.68 |
| *Micrococcus luteus* AHU 1427 | 0.22 |
| *Mycoplana bullata* IFO 13267 | 0.55 |
| *Mycoplana dimorpha* IFO 13213 | 0.98 |
| *Rhodopseudomonas spheroidas* ATCC 21286 | 0.11 |

* 2.66 mg of the compound is produced in the yield of 100%.

Example 16

Preparation of Compound (A) (Alternative method):

In this Example, *Clostridium acetobutylicum* IFO 3346 is inoculated in 100 ml of Potato Dextrose Broth (product of DIFCO Lab.). The fermentor is sealed after the air is substituted with sterilized nitrogen and culturing is carried out at a temperature of 30°C for 48 hours. Cell bodies are recovered

and washed with physiological saline solution. The cell is suspended in 2 ml of 1/30 M potassium phosphate buffer (pH 6.5). The same reaction as in Example 15 is repeated to obtain 0.12 mg of Compound (A).

Example 17

Preparation of (6R, 7S)7 - (R) - phenylglycinamido - 3 - chloro - 1 - azabicyclo[4,2,0]oct - 2 - en - 8 - on - 2 - carboxylic acid (alternative method):

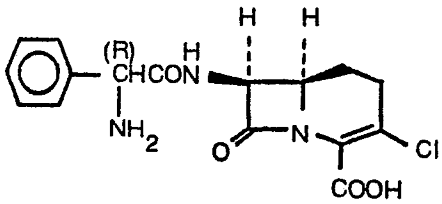

17—1) Preparation of cell suspension

a) Cultivation of a microorganism having an ability of optically selective acylation.

As a seed strain, *Pseudomonas melanogenum* ATCC 17808 [Biological properties are described in Journal of the Agricultural Chemical Society of Japan *37*, 71 (1963)] is used.

As the seed medium, an aqueous solution containing 1% polypeptone, 1% yeast extract, 0.5% meat extract, 0.5% sodium glutamate and 0.25% sodium chloride and adjusted to a pH of 7.0 with 5N—NaOH is used. One loopful seed strain is inoculated into 10 ml the seed medium in a 50 ml test tube and culturing is carried out at a temperature of 30°C for 24 hours. The whole amount of the seed medium is put into 300 ml of the culture medium in a 2 l Erlenmeyer flask and culturing is carried out at a temperature of 30°C. The composition of the culture medium is the same as that of the seed medium.

b) Preparation of cell suspension

After culturing for 24 hours, cell bodies are recovered from the culture broth by centrifugation and washed 2 times with 50 ml of 0.9% saline solution. The cells are suspended at a concentration of 20 mg/ml by dry weight in 1/30 M phosphate buffer (pH 6.5).

17—2) Preparation of a substrate solution

200 mg of the trifluoroacetate of (±) - cis - 7 - amino - 3 - chloro - 1 - azabicyclo-[4,2,0]oct - 2 - en - 8 - on - 2 - carboxylic acid (starting compound a) obtained as in Reference Example 8 and 800 mg of the hydrochloride of D-phenylglycine methylester (starting compound b) are added to 9 ml of 1/30 M potassium phosphate buffer (pH 6.5). 5N—KOH is added in a small portion and the mixture is again adjusted to a pH of 6.5 to dissolve the two starting compounds. Finally, deionized water is added to make 10 ml of solution.

17—3) Enzyme reaction

In this step 10 ml of the disrupted cell suspension is added to 10 ml of the substrate solution and enzyme reaction is carried out at a temperature of 30°C for 1.5 hours. The reaction is monitored by high speed liquid chromatography using TRI ROTAR and Prepack column Nucleosil 10C18.

Elution is carried out with 7% methanol-0.2 M $KH_2PO_4$ solution. Reaction reaches maximum in a yield of 90% to the starting compound in 1.5 hours.

17—4) Isolation and purification of the desired compound:

After the completion of reaction, cell bodies are removed from the reaction solution by centrifugation. The supernatant is concentrated under reduced pressure and charged on a column (1.6 cm width, 50 cm height) packed with Diaion HP—10.

After adding 200 ml of deionized water, elution is carried out with 25% aqueous methanol solution. Then, the fractions containing the desired compound are concentrated under reduced pressure to make 5 ml of concentrate. The concentrate is charged on a column (1.6 cm width, 64.5 cm height) packed with 130 ml of Sephadex—LH20 and elution is carried out with a solvent of water and methanol (50:50). The desired compound is eluted in fractions from 55 ml to 75 ml. The fractions are concentrated under reduced pressure and lyophilized to obtain 78 mg of a white powder. Properties of the product coincide with the less polar isomer (C) in Example 12.

Antibacterial activities of the compound obtained in Example 17 are shown in Table 2 as compound (a). Heart Infusion Agar Dilution Method (pH 7.2) is used. Cefazolin is used as a control. From the antimicrobial activity, absolute configuration of this compound is assumed to be (6R, 7S).

Example 18

Preparation of (6R, 7S) 7 - [(R) - 2 - p - hydroxyphenyl - 2 - aminoacetamido - 3 - chloro - 1 - azabicyclo[4,2,0]oct - 2 - en - 8 - on - 2 - carboxylic acid:

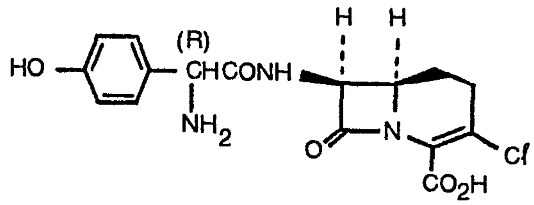

18—1) Preparation of cell suspension

Cultivation of a microorganism

The same procedure as in Example 17—1) is repeated except that as the seed strain, *Xanthomonas citri* IFO 3835 [Biological properties are described in Bergey's Manual of Determinative Bacteriology VI p. 156 (1948)] is used.

18—2) Preparation of substrate solution

200 mg of the trifluoroacetate of (±) - cis - 7β - amino - 3 - chloro - 1 - azabicyclo-[4,2,0]oct - 2 - en - 8 - on - 2 - carboxylic acid obtained as in Reference Example 8 and 800 mg of the hydrochloride of D-p-hydroxyphenylglycine methylester are added to 9 ml of 1/30 M potassium phosphate buffer (pH 6.5). 5N—KOH is added in small portions and the mixture is again adjusted to a pH of 6.5 to dissolve the starting compounds. Finally, deionized water is added to make 10 ml of solution.

18—3) Enzyme reaction and 18—4) Isolation and purification of the desired compound

After the completion of the reaction, cells are removed by centrifugation and the resulting filtrate is concentrated under reduced pressure. The concentrate is charged on a column (1.6 cm width, 50 cm height) packed with 100 ml of Diaion HP—20AG (100 to 200 mesh, product of Mitsubishi Kasei Kogyo Co., Ltd.). Then 150 ml of water and 150 ml of 10% aqueous methanol are passed through the column and elution is carried out with 20% aqueous methanol. Fractions (210 to 750 ml) of eluted 20% aqueous methanol are combined and concentrated under reduced pressure. The concentrate is charged on a column (0.88 cm width, 70 cm height) packed with 43 ml of Sephadex LH20 and elution is carried out with a mixture of water and methanol (1:1, by volume). Fractions (28 to 34 ml) are combined and concentrated under reduced pressure to remove methanol. The resulting residue is lyophilized to obtain 47 mg of a white powder. The product is identified as the desired compound based on the following properties.

$[\alpha]_D^{20°} = +44.0°$ [c=0.25, 1 M phosphate buffer (pH 7.0)]

IR $\nu_{max}^{KBr}$ ($cm^{-1}$): 1765, 1695, 1615

NMR (100 M, $D_2O$—DSS) δ: 7.36 (2H, d, J=8.8Hz), 6.96 (2H, d, J=8.9Hz), 5.36 (1H, d, J=4.6Hz), 5.11 (1H, s), 3.81—4.00 (1H, m), 2.42—2.58 (2H, m), 1.59—1.77 (1H, m), 1.17—1.48 (1H, m)

Antimicrobial activity of the compound obtained in this example is shown in Table 2 as compound (b).

Example 19

Preparation of (6R, 7S) 7 - (R) - 2 - phenylglycinamido - 3 - chloro - 1 - azabicyclo-[4,2,0]oct - 2 - en - 8 - on - 2 - carboxylic acid (Alternative method):

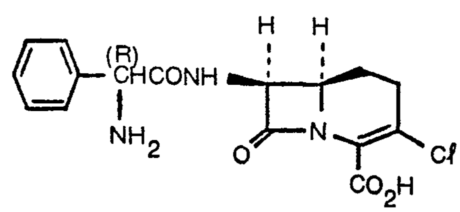

19—1) Preparation of cell suspension

a) Cultivation of a microorganism having an ability of optically selective acylation

As a seed strain, *Pseudomonas melanogenum* ATCC 17808 [Biological properties are described in Journal of the Agricultural Chemical Society of Japan, *37,* 71 (1963)] is used.

As a seed medium, an aqueous solution containing 1% polypeptone, 1% yeast extract, 0.5% meat extract, 0.5% sodium glutamate and 0.25% sodium chloride and adjusted at a pH of 7.0 with 5N—NaOH is used. One loopful seed strain is inoculated into 10 ml of the seed medium in a 50 ml test

tube and culturing is carried out at a temperature of 30°C for 24 hours. The whole amount of the seed medium is put into 300 ml of the culture medium in a 2 l Erlenmeyer flask and culturing is carried out with shaking at a temperature of 30°C. The composition of the culture medium is the same as that of the seed medium.

b) Preparation of cell suspension

After culturing for 24 hours, cell bodies are recovered from the culture broth by centrifugation and washed 2 times with 50 ml of 0.9% saline solution. The concentrate is charged on a column (1.6 cm width, 64.5 cm height) packed with 130 ml of Sephadex—LH20 (Pharmacia and elution is carried out with a mixture of water and methanol (50:50). The desired compound is eluted in fractions 55 ml to 75 ml. The fractions are concentrated under reduced pressure and the residue is lyophilized to obtain 12.8 mg of a white powder. Properties of the product coincide with those of the product obtained in Example 17. The cells are suspended at a concentration of 20 mg/ml by dry weight in 1/30 M phosphate buffer (pH 6.5).

19—2) Preparation of substrate solution

100 mg of (—) - cis - 7 - amino - 3 - chloro - 1 - azabicyclo[4,2,0]oct - 2 - en - 8 - on - 2 - carboxylic acid (Starting compound a) obtained as in Reference Example 9 and 800 mg of the hydrochloride of D-phenylglycine methylester are added to 9 ml of 1/30 M potassium phosphate buffer (pH 6.5). 5N—KOH is added in small portions and the mixture is again adjusted to a pH of 6.5 to dissolve the two starting compounds. Finally, deionized water is added to make 10 ml of solution.

19—3) Enzyme reaction

In this step, 10 ml of the cell suspension is added to 10 ml of the substrate solution and enzyme reaction is carried out at a temperature of 30°C for 1.5 hours. The reaction is monitored by high speed liquid chromatography using TRIROTAR and Prepack column Nucleosil 10C18. Elution is carried out with 7% methanol—0.2 M $KH_2PO_4$ solution. Reaction reaches maximum in 1.5 hours. Yield to the starting compound (a) is 90%.

19—4) Isolation and purification of the desired compound

After the completion of reaction, cell bodies are removed from the reaction solution by centrifugation. The supernatent is concentrated under reduced pressure and charged on a column (1.6 cm width, 50 cm height) packed with 100 ml of Diaion HP—10. After adding 200 ml of deionized water, elution is carried out with 25% aqueous methanol solution. The fractions containing the desired compound are concentrated under reduced pressure to make 5 ml of concentrate. The concentrate is charged on a column (1.6 cm width, 64.5 cm height) packed with 130 ml of Sephadex LH 20 (product of Pharmacia Fine Chemicals Inc.) and elution is carried out with a mixture of water and methanol (50:50). The desired compound is eluted in fractions of 55 ml to 75 ml. The fractions are concentrated under reduced pressure and lyophilized to obtain 128 mg of a white powder. Properties of the product coincide with those of the product obtained in Example 17.

## Example 20

Preparation of (6R, 7S) 7 - [(R) - 2 - phenyl - 2 - aminoacetamido] - 3 - chloro - 1 - azabicyclo[4,2,0]oct - 2 - en - 8 - on - 2 - carboxylic acid (Alternative method):

Example 17—1) is repeated except that as the seed strain *Kluyvera citrophila* ATCC 21285 [Biological properties are described in J. General Applied Microbiology *3,* 27—31 (1957)] is used. After 24 hours of the main cultivation, cells are recovered from the culture broth by centrifugation and a cell suspension is obtained as in the same treatment as Example 17—1). Preparation of a substrate solution and enzyme reaction are carried out in the same manner as in Example 17—2) and 17—3), respectively, except that reaction is carried out for 20 hours. Purification is carried out by column chromatography using HP—10 and Sephadex LH20 as in Example 17—4). Fractions containing the desired compound are lyophilized to obtain 86 mg of a white powder. Properties of the product coincide with those of the compound in Example 17.

## Reference Example 1

Antimicrobial activities of the compounds obtained in Examples 1 and 2 are as follows. Heart Infusion Agar Dilution Method (pH 7.2) is used. The cephalosporin compound having the same acyl side chain corresponding to the dl-compound is used as a control.

A : The compound obtained in Example 1
A′: The dl-compound corresponding to the compound obtained in Example 1
B : The compound obtained in Example 2
B′: The dl-compound corresponding to the compound obtained in Example 2

C : Cephalosporin compound represented by the following formula.

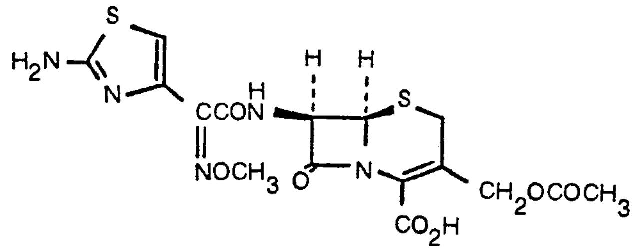

TABLE 4

| Microorganism | MIC (μg/ml) | | | | |
|---|---|---|---|---|---|
| | A | A′ | B | B′ | C |
| Staphylococcus aureus 209–P | 3.12 | 12.5 | 1.56 | 6.25 | 0.78 |
| Staphylococcus aureus Smith | 6.25 | 25 | 6.25 | 12.5 | 1.56 |
| Staphylococcus epidermidis | 12.5 | 25 | 3.12 | 12.5 | 1.56 |
| Escherichia coli NIHJC–2 | 0.02 | 0.05 | 0.02 | 0.05 | 0.1 |
| Escherichia coli GN2411–5 | 0.01 | 0.05 | ≤0.01 | 0.02 | 0.05 |
| Escherichia coli Juhl | 0.02 | 0.1 | 0.02 | 0.05 | 0.05 |
| Klebsiella pneumoniae 8045 | ≤0.006 | ≤0.006 | ≤0.01 | 0.01 | ≤0.01 |
| Klebsiella pneumoniae Y–60 | 0.02 | 0.05 | 0.02 | 0.05 | 0.05 |
| Serratia marcescens T–26 | 0.2 | 0.78 | 0.4 | 0.78 | 0.78 |
| Serratia marcescens T–55 | 0.02 | 0.1 | 0.05 | 0.2 | 0.1 |
| Proteus mirabilis 1287 | 0.01 | 0.02 | ≤0.01 | 0.01 | 0.02 |
| Proteus vulgaris 6897 | ≤0.006 | 0.01 | ≤0.01 | 0.01 | ≤0.01 |
| Proteus morganii KY 4298 | 0.05 | 0.1 | 0.02 | 0.1 | 0.05 |
| Proteus rettgeri KY 4289 | ≤0.006 | ≤0.006 | ≤0.01 | 0.01 | ≤0.01 |
| Pseudomonas aeruginosa #1 | 6.25 | 25 | 25 | 50 | 6.25 |
| Pseudomonas aeruginosa 145 | 50 | 50 | 100 | >100 | 50 |
| Pseudomonas putida 264 | 0.1 | 0.4 | 0.05 | 0.2 | 0.1 |

Reference Example 2

Preparation of (+)-cis-7-amino-1-azabicyclo[4,2,0]oct-2-en-8-on-2-carboxylic acid:

2—1) Preparation of disrupted cell suspension

1) Cultivation of a microorganism having an ability to effect optical selective deacylation

As the seed strain, *Kluyvera citrophila* ATCC 21285 [Biological properties are described in J. General Applied Microbiology *3,* 28—31 (1957)] is used.

As the seed medium, an aqueous solution containing 1% polypeptone, 1% yeast extract, 0.5% meat extract, 0.5% sodium glutamate and 0.25% sodium chloride and adjusted at a pH of 7.0 with 5N—NaOH is used. One loopful of the seed strain is inoculated into 10 ml of the seed medium in a

50 ml test tube and culturing is carried out at a temperature of 30°C for 24 hours. The whole of the seed broth is inoculated into 300 ml of the culture medium in a 2 l Erlenmeyer flask and culturing is carried out at a temperature of 30°C with shaking. The composition of the culture medium is the same as that of the seed medium.

2) Preparation of disrupted cell suspension

After culturing for 24 hours, the culture broth is subjected to centrifugation to obtain cell bodies. The cells are washed twice with 50 ml of 0.9% saline solution and suspended at a concentration of 40 mg/ml by dry weight in 1/30 M phosphate buffer solution. Then, 10 ml of the cell suspension is put in a 50 ml test tube and subjected to ultrasonic disintegration at 200 W for 2 minutes to obtain a disrupted cell suspension. In the treatment, ultrasonic disintegrator Model UR200P (product of Tomy Seiko Co., Ltd.) is used.

2—2) Preparation of a substrate solution

In this step, 200 mg of (±)-cis-7-phenylacetamid-1-azabicyclo[4,2,0]oct-2-en-8-on-2-carboxylic acid obtained as in DE—A—2911787 is added to 9 ml of 1/30 phosphate buffer (pH 6.5). Since the compound is not dissolved. 2N—NaOH is added in a small portion and the mixture is again adjusted to a pH of 6.5 to dissolve the compound. Finally deionized water is added to make 10 ml of solution.

2—3) Enzyme reaction

In this step, 10 ml of the disrupted cell suspension mentioned above is added to 10 ml of the substrate solution and an enzyme reaction is carried out at a temperature of 30°C for 80 minutes. Time course of the reaction is illustrated in Table 5.

TABLE 5

| Reaction period (minutes) | The amount of Compound (III—1) produced (mg/ml) | Yield (Mol ratio, %) |
|---|---|---|
| 10 | 2.0 | 33 |
| 20 | 2.6 | 43 |
| 40 | 2.9 | 48 |
| 60 | 3.0 | 50 |
| 80 | 3.0 | 50 |

As apparent from the Table 5, the reaction and yield are stationary since the conversion ratio of the mixture of the optically active isomers reaches 50% (mol ratio).

2—4) Isolation and Purification of the desired compound

After the completion of the reaction, cells are removed by centrifugation from the reaction solution. The supernatant is adjusted at a pH of 3.0 with 2N-hydrochloric acid and charged on a column (2.6 cm width, 51 cm height) packed with 270 ml of Diaion HP—10.

Elution is carried out with deionized water and the eluate is collected in 5 ml fractions. The desired compound is eluted in fractions from 280 ml to 315 ml. The fractions are concentrated under reduced pressure, lyophilized and dissolved in a small amount of a mixture of water and methanol (50:50 by volume, the same shall apply hereinafter). The solution is charged on a column (1.6 cm width, 64.5 cm height) packed with 130 ml of Sephadex LH 20 (Farmacia Fine Chemicals Inc.). Elution is carried out with a mixture of water and methanol (50:50). The eluate is collected in 5 ml fractions. Fractions from 65 ml to 85 ml are combined and concentrated under reduced pressure to remove methanol. Then, the residue is lyophilized to obtain 48 mg of a white powder. Properties of the product are as follows.

IR (KBr) $\nu_{max}^{cm^{-1}}$: 1800, 1790, 1775, 1640, 1620

NMR (100 M $D_2O$—DSS) $\delta$: 6.46 (1H, dd, J=3.5, 4.7Hz), 4.88 (1H, d, J=5.2Hz), 4.06 (1H, m), 2.5—1.5 (4H, m)

It is revealed that the compound has one mole of hydrochloric acid and water. The properties of the compound coincide well with those of the corresponding dl-compound. The value of optical rotation is $[\alpha]_D^{15°}$ = +48° [c=0.5, in 1 M phosphate buffer solution (pH 7.0)] which conicides well with the value in Reference Example 3 below, $[\alpha]_D^{15°}$ = 48.5° [c=0.5, in 1 M phosphate buffer solution (pH 7.0)].

The compound shows a ninhydrin positive single spot at an Rf value of 0.22 on silica gel thin layer chromatography [thin layer plate Merck Art 5721 (product of E. Merck & Co.), solvent for development, isopropanol:acetic acid:water = 4:1:1]. The Rf value coincides with that of the optically inactive dl-compound.

Reference Example 3

Preparation of (+)-cis-7-amino-1-azabicyclo[4,2,0]oct-2-en-8-on-2-carboxylic acid (Alternative method)

3—1) Preparation of disrupted cell suspension

The same procedure as in Reference Example 2—1) is repeated.

3—2) Preparation of a substrate solution

In this step, 100 mg of (+)-cis-7-[(R)-2-phenyl-2-aminoacetamido]-1-azabicyclo[4,2,0]oct-2-en-8-on-2-carboxylic acid obtained as in DE—A—2911787 is dissolved in 5 ml of 1/30 M phosphate buffer solution (pH 6.5).

3—3) Enzyme reaction

In this step, 5 ml of the disrupted cell suspension mentioned above is added to 5 ml of the substrate solution and enzyme reaction is carried out at 30°C for 24 hours.

3—4) Isolation and Purification

In this step, 46 mg of a white powder is obtained by a similar method as in Reference Example 2—4). Properties of the compound coincide well with those of the compound obtained in Reference Example 2.

$[\alpha]_D^{15°} = 48.5°$ [c=0.5, in 1 M phosphate buffer solution (pH 7.0)]

Reference Example 4

Preparation of (—) - cis - 7$\beta$ - amino - 4$\alpha$ - methyl - 1 - azabicyclo[4,2,0]oct - 2 - en - 8 - on - 2 - carboxylic acid:

4—1) Preparation of disrupted cell suspension

A similar procedure as in Reference Example 2—1) is repeated.

4—2) Preparation of a substrate solution

A similar procedure as in Reference Example 2—2) is repeated except that (±-cis-7$\beta$-phenylacetamid-4$\alpha$-methyl-1-azabicyclo[4,2,0]oct-2-en-8-on-carboxylic acid obtained as in DE—A—2911787 is used.

4—3) Enzyme Reaction

A similar procedure as in Reference Example 2—3) is repeated except that the disrupted cell suspension and the substrate solution obtained in 4—1) and 4—2) are used. Reaction ratio becomes stationary in one hour. The reaction is continued for 120 minutes. The yield is 50% (mol ratio) of the mixture of optically active compounds.

4—4) Isolation and purification of the desired compound

An almost similar procedure as in Reference Example 2—4) is repeated. After the completion of reaction, cells are removed by centrifugation from the reaction solution. The supernatant is charged on a column (2.5 cm width, 46 cm high) packed with 220 ml of Diaion HP—10. Elution is carried out with deionized water and the eluate is collected in 5 ml fractions. The desired compound is eluted in fractions from 200 ml to 270 ml. The fractions are concentrated under reduced pressure, lyophilized, and dissolved in a small amount of water and methanol (50:50). The solution is charged on a column (1.6 cm width, 64.5 cm height) packed with 130 ml of Sephadex LH—20 and elution is carried out with a mixture of water and methanol (50:50). The eluate is collected in 5 ml fractions. The fractions from 65 ml to 80 ml are combined and concentrated to remove methanol. Then, the residue is lyophilized to obtain 30.5 mg of a white powder. Properties of the compound are as follows.

IR (KBr) $\nu_{max}^{cm^{-1}}$: 1800, 1770(sh), 1760(sh), 1740, 1680, 1630

NMR (100 M $D_2O$—DSS)$\delta$: 6.16 (1H, d, J=5.1 Hz), 4.52 (1H, d, J=4.9Hz), 3.86 (1H, m), 2.64 (1H, m), 1.9—1.4 (2H, m), 1.10 (3H, d, J=7.3Hz)

It is revealed that the compound is a potassium salt having 2 moles of water. The properties above coincide well with those of the corresponding dl compound. The compound shows a ninhydrin positive single spot at Rf = 0.33 on a silica gel thin layer chromatography (the same silica gel as in Example 1 is used). The Rf value coincides with that of the optical inactive dl-compound.

Optical rotation $[\alpha]_D^{15°} = -30°$ (c=0.5, in 1 M phosphate buffer solution). The value coincides well with that in Reference Example 4, $[\alpha]_D^{15°} = -30.8°$ [c=0.5, in 1 M phosphate buffer solution (pH 7.0)].

Reference Example 5

Preparation of (−) - cis - 7β - amino - 4α - methyl - 1 - azabicyclo[4,2,0]oct - 2 - en - 8 - on - 2 - carboxylic acid (Alternative method)

5—1) Preparation of disrupted cell suspension

A similar procedure as in Reference Example 4—1) is repeated.

5—2) Preparation of a substrate solution

In this step, 100 mg of (+)-cis-7β-[(R)-2-phenyl-2-aminoacetamido]-4α-methyl-1-azabicyclo-[4,2,0]oct-2-en-8-on-2-carboxylic acid obtained in DE—A—2911787 is dissolved in 5 ml of 1/30 M phosphate buffer solution.

5—3) Enzyme reaction

In this step, 5 ml of the disrupted cell suspension described above is added to 5 ml of the substrate solution and enzyme reaction is carried out at a temperature of 30°C for 24 hours.

5—4) Isolation and Purification of the desired product

A similar procedure as in Reference Example 4—4) is repeated to obtain 55 mg of a white powder. Properties of the compound coincide well with those in Reference Example 4.

Optical rotation $[\alpha]_D^{15°} = -30.8°$ [c=0.5, in 1 M phosphate buffer solution (pH 7.0)].

Reference Example 6

Preparation of (±) - cis - 7 - azido - 3 - chloro - 2 - t - butyloxycarbonyl - 1 - azabicyclo-[4,2,0]oct - 2 - en - 8 - one:

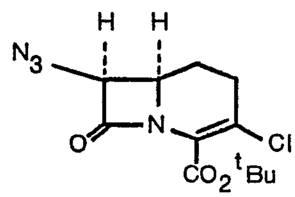

The present compound is prepared according to the following processes a), b), c) and d).

6—a) Preparation of (±) - cis - 7 - azido - 3 - phenylthio - 1 - azabicyclo[4,2,0]octane - 8 - on - 2 - carboxylic acid, t-butylester:

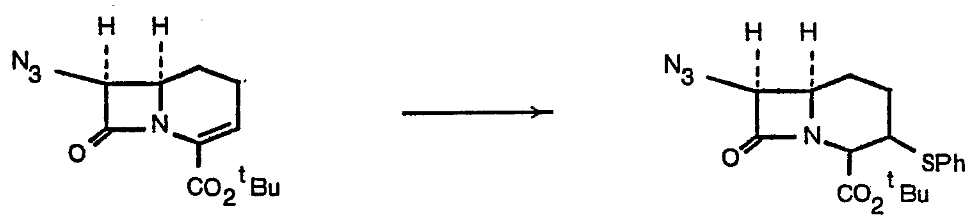

In this Example, 528 mg (2 m mole) of (±)-cis-7-azido-1-azabicyclo[4,2,0]oct-2-en-8-on-2-carboxylic acid, t-butylester prepared as in the method described in Japanese Published Unexamined Patent Application No. 128591/79 is dissolved in 15 ml of anhydrous benzene and 0.2 ml (2 m mole) of thiophenol and 0.2 ml (2 m mole) of piperidine are added. The mixture is stirred at room temperature for 2 hours. After the completion of reaction, the reaction solution is washed with 10% citric acid and saturated saline solution and dried with anhydrous sodium sulfate. The solvent is removed by distillation under reduced pressure and the obtained oily residue is purified by silica gel column chromatography using 30 g of silica gel and a solvent of ethyl acetate and n-hexane (1:4) to obtain 720 mg (96.3%) of the desired compound.

Melting point: 77.5—78.0°C

IR $\nu_{max}^{KBr}$ ($cm^{-1}$): 2110, 1765, 1745

NMR ($CDCl_3$) δ: 7.28—7.60 (5H, m), 4.78 (1H, d, J=5Hz), 4.33 (1H, s), 3.78—3.98 (1H, m), 3.81 (1H, s), 1.50—2.34 (4H, m), 1.42 (9H, s)

6—b) Preparation of (±) - cis - 7 - azido - 3 - phenylsulfinyl - 1 - azabicyclo[4,2,0]octane - 8 - on - 2 - carboxylic acid, t-butylester:

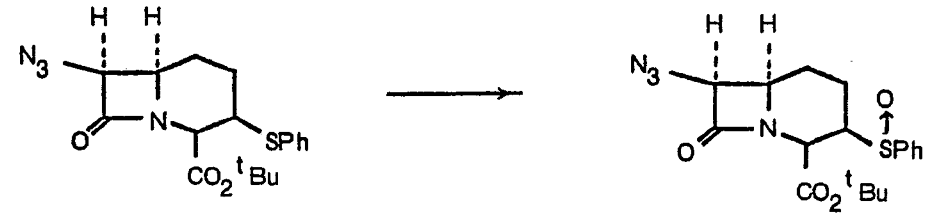

In this Example, 480 mg (1.28 m mole) of 3-phenylthio compound obtained as in Reference Example 6—a) is dissolved in 50 ml of anhydrous chloroform and 240 mg (1.41 m mole) of m-chloroperbenzoic acid under ice cooling. The mixture is allowed to react with stirring under ice cooling for 30 minutes. The reaction mixture is washed with saturated sodium bicarbonate and saturated sodium chloride and dried with anhydrous sodium sulfate. The solvent is removed by distillation under reduced pressure to obtain 500 mg (99.9%) of the desired compound.

Melting point: 95.5—96.5°C

IR $\nu_{max}^{KBr}$ ($cm^{-1}$): 2120, 2100, 1780, 1735, 1035

NMR ($CDCl_3$) $\delta$: 7.55—7.91 (5H, m), 4.87 (1H, d, J=4.6Hz), 4.05 (1H, s) 3.90—4.10 (1H, m), 3.10 (1H, s), 1.70—2.84 (4H, m), 1.30 (9H, s)

6—c) Preparation of (±) - cis - 7 - azido - 3 - chloro - 3 - phenylsulfinyl - 1 - azabicyclo-[4,2,0]octane - 8 - on - 2 - carboxylic acid, t-butylester:

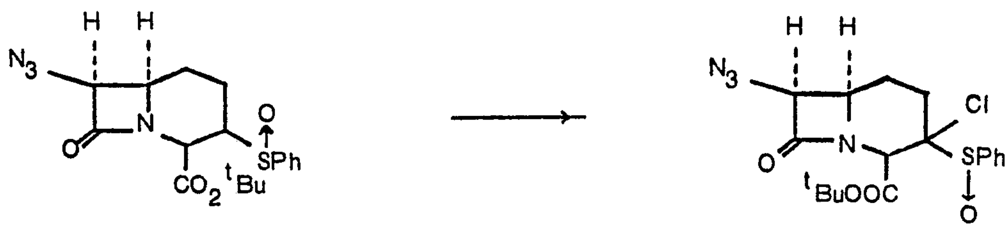

In this Example, 109 mg of sulfoxide compound obtained as in Reference Example 6—b) is dissolved in 1 ml of anhydrous methylene chloride and 23.5 mg (0.42 m mole) of calcium oxide is added. After adding 27 $\mu$l (0.34 m mole of sulfinyl chloride, the mixture is allowed to react with stirring under ice cooling for one hour. The reaction mixture is washed with 10% citric acid, saturated sodium bicarbonate and saturated sodium chloride solution and dried with anhydrous sodium sulfate. The solvent is removed by distillation and the obtained oily residue is subjected to silica gel chromatography using 5 g of silica gel and a solvent of ethyl acetate and n-hexane (1:5) to obtain 66.5 mg (56.1%) of the purified desired oily compound.

IR $\nu_{max}^{CHCl_3}$ ($cm^{-1}$): 2120, 1770, 1735, 1055

NMR ($CDCL_3$) $\delta$: 7.53—8.00 (5H, m), 4.90 (1H, d, J=5Hz), 4.43 (1H, s), 4.15—4.35 (1H, m), 1.83—2.85 (4H, m), 1.38 (9H, s)

6—d) Preparation of (±) - cis - 7 - azido - 3 - chloro - 1 - azabicyclo[4,2,0]oct - 2 - en - 8 - on - 2 - carboxylic acid, t-butylester:

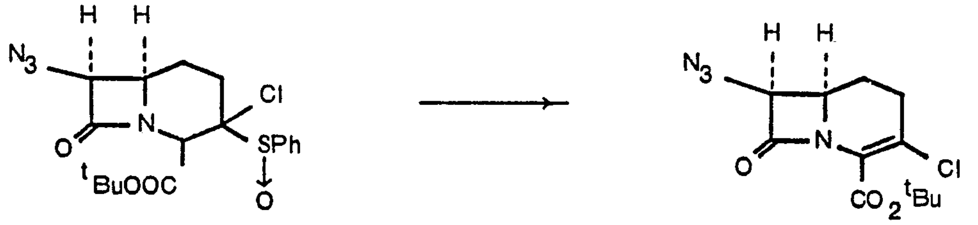

In this Example, 1.3 g (3.06 m mole) of 3-chloro-3-phenylsulfinyl compound obtained as in Reference Example 6—c) is heated under reflux with 100 ml of carbon tetrachloride for 6 hours. After the completion of reaction, the reaction solution is subjected to distillation under reduced pressure to remove the solvent. The obtained residue is subjected to purification by silica gel chromatography using

100 g of silica gel and a solvent of ethyl acetate and n-hexane (1:5) to obtain 596 mg (65.2%) of the desired compound.

Melting point: 96—97°C
IR$\nu_{max}^{KBr}$ ($cm^{-1}$): 2120, 1765, 1735, 1630
PMP ($CDCl_3$) $\delta$: 4.93 (1H, d, J=5Hz), 3.72—3.92 (1H, m), 2.56—2.70 (2H, m), 1.86—2.32 (2H, m), 1.55 (9H, s)
CMR ($CDCl_3$) $\delta$: 127.7, 125.0

Reference Example 7

Preparation of (±) - cis - 7 - amino - 3 - chloro - 1 - azabicyclo[4,2,0]oct - 2 - en - 8 - on - 2 - carboxylic acid, t-butylester:

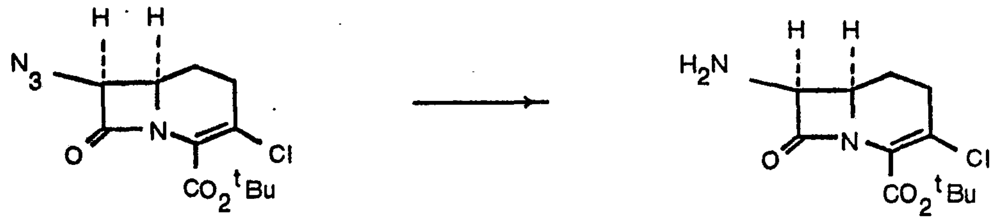

In this Example, 350 mg (1.17 m mole) of the azido compound obtained as in Reference Example 6 is dissolved in 20 ml of ethanol and 1.2 ml of 1N-hydrochloric acid and 70 mg of 10% palladium-carbon. Hydrogen gas is passed through the mixture at room temperature and atmospheric pressure for 3 hours and palladium-carbon is removed by filtration. The filtrate is concentrated under reduced pressure to obtain a solid. The solid is dissolved in water and washed with ether. After adding sodium bicarbonate to make weakly alkaline, the water layer is extracted with ethyl acetate. The ethyl acetate layer is washed with saturated saline solution and dried with anhydrous sodium sulfate. The solvent is removed by distillation under reduced pressure to obtain 218.4 mg (68.4%) of a powder of the desired compound.

Melting point: 102.5—104.5°C
IR$\nu_{max}^{KBr}$ ($cm^{-1}$): 1770, 1720, 1620
NMR ($CDCl_3$) $\delta$: 4.43 (1H, d, J=5Hz), 3.52—3.90 (1H, m), 2.52—2.72 (2H, m), 2.22 (2H, br), 1.87—2.17 (2H, m), 1.55 (9H, s).

Reference Example 8

Preparation of the trifluoroacetate of (±)-cis-7-amino-3-chloro-1-azabicyclo[4,2,0]oct-2-en-8-on-2-carboxylic acid:

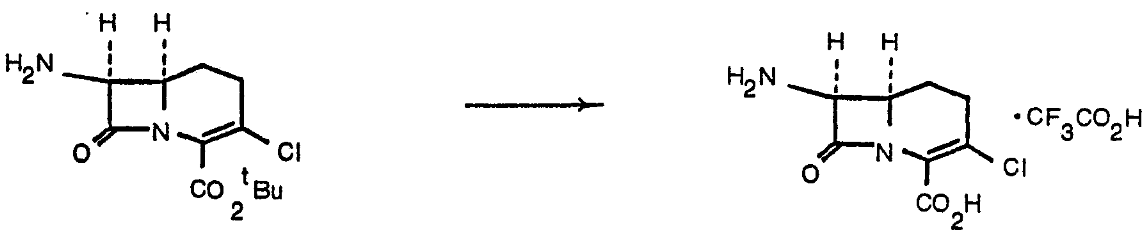

In this Example, 102.2 mg (0.31 m mole) of the amino-ester compound obtained as in Reference Example 7 and 1 ml of trifluoroacetic acid is added under ice cooling. The mixture is stirred at room temperature for 30 minutes. The solvent is removed by distillation under reduced pressure to obtain an oily residue. To the residue is added ether and the resulting powder is recovered by filtration. The powder is lyophilized to obtain 75.5 mg (60.9%) of the desired compound.

Melting point: 208—220°C (dec.)
IR$\nu_{max}^{KBr}$ ($cm^{-1}$): 1795, 1630

Reference Example 9

Preparation of (—) - cis - 7 - amino - 3 - chloro - 1 - azabicyclo[4,2,0]oct - 2 - en - 8 - on - 2 - carboxylic acid:

9—1) Preparation of disrupted cell suspension

1) Cultivation of a microorganism having an ability to effect optically selective deacylation.

As the seed stain, *Kluyvera citrophila* ATCC 21285 [Biological properties are described in J. General Applied Microbiology *3*, 28—31 (1957)] is used.

As the seed medium, an aqueous solution containing 1% polypeptone, 1% yeast extract, 0.5% meat extract, 0.5% sodium glutamate and 0.25% sodium chloride and adjusted at a pH of 7.0 with 5N—NaOH is used. One loopful seed strain is inoculated into 10 ml of the seed medium in a 50 ml test tube and culturing is carried out at a temperature of 30°C for 24 hours. The whole of the seed broth is inoculated into 300 ml of the culture medium in a 2 l Erlenmeyer flask and culturing is carried out at a temperature of 30°C with shaking. The composition of the main culture medium is the same as that of the seed medium.

2) Preparation of disrupted cell suspension

After culturing for 24 hours, the culture broth is subjected to centrifugation to obtain cell bodies. The cells are washed twice with 50 ml of 0.9% saline solution and suspended at a concentration of 40 mg/ml by dry weight in 1/30 M phosphate buffer solution (pH 8.0).

9—2) Preparation of a substrate solution

In this step, 200 mg of (±)-cis-7-phenylacetamido-3-chloro-1-azabicyclo[4,2,0]oct-2-en-8-on-2-carboxylic acid obtained as in Example 13 is added to 9 ml of 1/30 M phosphate buffer (pH 8.0). Since the compound is not dissolved, 2N—NaOH is added in a small portion and the mixture is again adjusted to a pH of 8.0 to dissolve the compound. Finally, deionized water is added to make 10 ml of solution.

9—3) Enzyme reaction

In this step, 10 ml of the disrupted cell suspension mentioned above is added to 10 ml of the substrate solution and enzyme reaction is carried out at a temperature of 40°C for 80 minutes. Time course of the reaction is illustrated in Table 6.

TABLE 6

| Reaction period (minutes) | The amount of Compound (III—1) produced (mg/ml) | Yield (Mol ratio, %) |
|---|---|---|
| 10 | 1.3 | 20 |
| 20 | 1.8 | 28 |
| 40 | 2.0 | 31 |
| 60 | 2.3 | 36 |
| 80 | 2.4 | 37 |

9—4) Isolation and Purification of the desired compound

After the completion of the reaction, cells are removed by centrifugation from the reaction solution. The supernatant is concentrated under reduced pressure to make 5 ml of solution. The solution is charged on a column (1.75 cm width, 42 cm height) packed with Diaion HP—10. Elution is carried out with deionized water. The desired compound is eluted in fractions from 90 ml to 120 ml. The fractions are concentrated under reduced pressure, to make 2 ml of solution and the solution is adjusted to pH 3.5 with 1N-hydrochloric acid to deposit crystals.

The crystals are recovered by filtration, washed with a small amount of methanol and dried to obtain 38 mg of a white powder. Properties of the product are as follows.

IR (KBr) $\nu_{max}^{cm^{-1}}$: 3200, 1800, 1790(sh), 1640(sh), 1630, 1555
NMR (100 M $D_2O$—DSS)$\delta$: 4.47 (1H, d, J=5.1Hz), 3.88 (1H, m), 2.64 (2H, m), 1.93 (2H, m)
Optical rotation $[\alpha]_D^{25}=-2.7°$ (c=0.24, 1 M phosphate buffer, pH 7.0)

Reference Example 10

Preparation of (—) - 7$\beta$ - amino - 4$\alpha$ - methyl - 1 - azabicyclo[4,2,0]oct - 2 - en - 8 - on - 2 - carboxylic acid:

10—1) Preparation of disrupted cell suspension

A similar procedure as in Reference Example 2—1) is repeated.

10—2) Preparation of a substrate solution

A similar procedure as in Reference Example 2—2) is repeated except that (±)-cis-7$\beta$-phenylacetamido-4$\alpha$-methyl - 1 - azabicyclo[4,2,0]oct - 2 - en - 8 - on - 2 - carboxylic acid obtained as in DE—A—2911787 is used.

10—3) Enzyme reaction

A similar procedure as in Reference Example 2—3) is repeated except that the disrupted cell suspension and the substrate solution obtained in 10—1) and 10—2) are used. Reaction ratio becomes stationary in one hour. The reaction is continued for 120 minutes. The yield is 50% (mol ratio) of the mixture of optically active compounds.

10—4) Isolation and purification of the desired compound

An almost similar procedure as in Reference Example 2—4) is repeated. After the completion of reaction, cells are removed by centrifugation from the reaction solution. The supernatant is charged on a column (2.5 cm width, 46 cm height) packed with 220 ml of Diaion HP—10. Elution is carried out with deionized water and the eluate is collected in 5 ml fractions. The desired compound is eluted in fractions from 200 ml to 270 ml. The fractions are concentrated under reduced pressure, lyophilized, and dissolved in a small amount of water and methanol (50:50). The solution is charged on a column (1.6 cm width, 64.5 cm height) packed with 130 ml of Sephadex LH—20 and elution is carried out with a mixture of water and methanol (50:50). The eluate is collected in 5 ml fractions. The fractions from 65 ml to 80 ml are combined and concentrated to remove methanol. Then, the residue is lyophilized to obtain 30.5 mg of a white powder. Properties of the compound are as follows.

IR (KBr) $\nu_{max}^{cm^{-1}}$: 1800, 1770(sh), 1760(sh), 1740, 1680, 1630

NMR (100 M $D_2O$—DSS) $\delta$: 6.16 (1H, d, J=5.1Hz), 4.52 (1H, d, J=4.9Hz), 3.86 (1H, m), 2.64 (1H, m) 1.9—1.4 (2H, m), 1.10 (3H, d, J=7.3Hz)

It is revealed that the compound is a potassium salt having 2 moles of water. The properties above coincide well with those of the corresponding dl-compound. The compound shows a ninhydrin positive single spot at Rf = 0.33 on a silica gel thin layer chromatography (the same silica gel as in Reference Example 2—4) is used). The Rf value coincides with that of the optically inactive dl-compound.

Optical rotation $[\alpha]_D^{15°}$ = −30° (c=0.5, in 1 M phosphate buffer solution). The value coincides well with that in Reference Example 11, $[\alpha]_D^{15°}$ = −30.8° [c=0,5, in 1 M phosphate buffer solution (pH 7.0)].

Reference Example 11

Preparation of (—) - 7β - amino - 4α - methyl - 1 - azabicyclo[4,2,0]oct - 2 - en - 8 - on - 2 - carboxylic acid (Alternative method).

11—1) Preparation of disrupted cell suspension

A similar procedure as in Reference Example 10—1) is repeated.

11—2) Preparation of a substrate solution

100 mg of (±) - cis - 7β - [(R) - 2 - phenyl - 2 - aminoacetamido] - 4α - methyl - 1 - azabicyclo[4,2,0]oct - 2 - en - 8 - on - 2 - carboxylic acid obtained as in DE—A—2911787 is dissolved in 5 ml of 1/30 M phosphate buffer solution (pH 6.5).

11—3 Enzyme reaction

As in Reference Example 2—3), 5 ml of the disrupted cell suspension described above is added to 5 ml of the substrate solution and enzyme reaction is carried out at a temperature of 30°C for 24 hours.

11—4) Isolation and Purification of the desired product

A similar procedure as in Reference Example 10—4) is repeated to obtain 55 mg of a white powder. Properties of the compound coincide well with those in Reference Example 10.

Optical rotation $[\alpha]_D^{15°}$ = −30.8° [c=0.5, in 1 M phosphate buffer solution (pH 7.0)].

**Claims**

1. Optically active acylated cephalosporin analogs represented by the general formula (I)

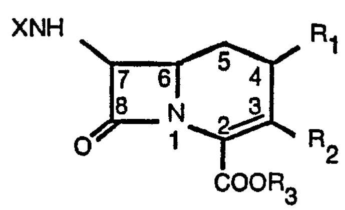

(I)

wherein $R_1$ represents a hydrogen or a lower alkyl group having 1 to 5 carbon atoms, $R_2$ represents a hydrogen or a halogen atom, $R_3$ represents a hydrogen or a protective group of carboxylic acid, X

represents an acyl group represented by the formula $X_1CO$ wherein $X_1$ represents the following two groups:

1) a group represented by the general formula

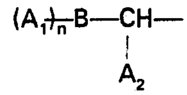

[wherein B represents an unsaturated six membered carbocycle which is selected from cyclohexenyl group, cyclohexadienyl group and phenyl group or a five or six membered heterocycle selected from furyl group, thienyl group, pyrrolyl group, thiazolyl group, oxazolyl group, isothiazolyl group, isoxazolyl group, imidazolyl group, pyrazolyl group, triazolyl group, tetrazolyl group, pyridinyl group, pyrimidinyl group, pyrazinyl group, pyrazinyl group, triazinyl group and 5,6-dihydro-1,4-dithiin-2-yl group, $A_1$ represents substituent(s) which is selected from hydroxyl group, a lower alkoxy group having 1 to 4 carbon atoms, a halo group, nitró group, amino group, aminomethyl group, and a methylsulfonamide group, n is a number from 0 to 5, and $A_2$ represents amino group, hydroxyl group, carboxyl group or sulfo group].

2) a group represented by the general formula

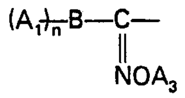

[wherein $A_1$, B and n have the same significance as defined above and $A_3$ represents hydrogen atom, a lower alkyl group having 1 to 6 carbon atoms, a lower alkenyl group having 2 to 6 carbon atoms, a lower alkynyl group having 2 to 6 carbon atoms, a cycloalkyl group having 3 to 6 carbon atoms or an aryl group, those groups being unsubstituted or substituted with suitable substituent(s) which is selected from carboxyl group, cyano group, a halo group, carbamoyl group and a lower alkoxycarbonyl group having 1 to 4 carbon atoms], and the hydrogens at the 6- and 7-positions have cis configuration and pharmaceutically acceptable salts thereof.

2. The compound according to claim 1 wherein $A_1$ is an amino group, B is a five membered heterocycle, $A_3$ is a lower alkyl group having 1 to 6 carbon atoms or substituted lower alkyl group having carboxyl group as substituent and $OA_3$ group has syn configuration.

3. The compound according to claim 2 wherein $R_2$ is a hydrogen, B is a 2-substituted thiazolyl group and $A_3$ is a methyl group.

4. The compound according to claim 3 wherein $R_1$ is a hydrogen.

5. The compound according to claim 4 wherein $R_3$ is a hydrogen, that is, (+) - cis - 7 - [2 - (2 - amino - 4 - thiazolyl) - 2 - syn - methoxyiminoacetamido] - 1 - azabicyclo[4,2,0]oct - 2 - en - 8 - on - 2 - carboxylic acid.

6. The compound according to claim 3 wherein $R_1$ is a methyl group.

7. The compound according to claim 6 wherein $R_3$ is a hydrogen.

8. The compound according to claim 7 wherein the methyl group at the 4-position has the same configuration as the hydrogens at the 6- and 7-positions, that is, (—) - cis - 7$\beta$ - [2 - (2 - amino - 4 - thiazolyl) - 2 - syn - methoxyiminoacetamido] - 4$\alpha$ - methyl - 1 - azabicyclo[4,2,0]oct - 2 - en - 8 - on - 2 - carboxylic acid.

9. The compound according to claim 1 wherein $A_1$ is a hydrogen or hydroxy group, B is a phenyl group and $A_2$ is an amino group.

10. The compound according to claim 9 wherein $R_1$ and $R_3$ are a hydrogen.

11. The compound according to claim 10 wherein $R_2$ is a chlorine atom.

12. The compound according to claim 11 wherein $A_1$ is a hydrogen atom and $A_2$ is an amino group, that is, (6R, 7S) 7 - (R) - phenylglycinamido - 3 - chloro - 1 - azabicyclo[4,2,0]oct - 2 - en - 8 - on - 2 - carboxylic acid.

13. The compound according to claim 11 wherein $A_1$ is p-hydroxy group and $A_2$ is an amino group, that is, (6R, 7S) 7 - (R) - p - hydroxyphenylglycinamido - 3 - chloro - 1 - azabicyclo[4,2,0]-oct - 2 - en - 8 - on - 2 - carboxylic acid.

14. The compound according to claim 9 wherein $R_2$ and $R_3$ are a hydrogen.

15. The compound according to claim 14 wherein $R_1$ is a hydrogen or a methyl group.

16. The compound according to claim 15 wherein $A_1$ is a hydrogen atom or p-hydroxy group and $A_2$ is an amino group.

17. The compound according to claim 16 wherein $R_1$ and $A_1$ are hydrogen atoms, that is, (6R, 7S) 7 - (R) - phenylglycinamido - 1 - azabicyclo[4,2,0]oct - 2 - en - 8 - on - 2 - carbocyclic acid.

18. The compound according to claim 16 wherein $R_1$ is a hydrogen and $A_1$ is a p-hydroxy group, that is, (6R, 7S) 7 - (R) - p - hydroxyphenylglycinamido - 1 - azabicyclo[4,2,0]oct - 2 - en - 8 - on - 2 - carboxylic acid.

19. The compound according to claim 15 wherein $R_1$ is a methyl group and $A_1$ is a hydrogen

atom, that is, (6R, 7S) 7 - (R) - phenylglycinamido - 4 - methyl - 1 - azabicyclo[4,2,0]oct - 2 - en - 8 - on - 2 - carboxylic acid.

20. The compound according to claim 15 wherein $R_1$ is a methyl group and $A_1$ is a p-hydroxy group, that is, (6R, 7S) 7 - (R) - p - hydroxyphenylglycinamido - 4 - methyl - 1 - azabicyclo-[4,2,0]oct - 2 - en - 8 - on - 2 - carboxylic acid.

21. A process for producing cephalosporin analogs represented by the general formula (I) according to Claim 1 and pharmaceutically acceptable salts thereof, which comprises acylating optically active cephalosporin analogs represented by the general formula (II):

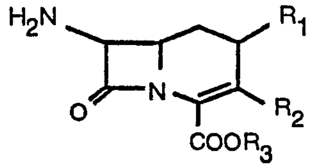

(II)

(wherein $R_1$, $R_2$ and $R_3$ have the same meaning as defined above and the hydrogens at the 6- and 7-positions have "cis" configuration) or a functionally equivalent compound with carboxylic acid represented by the general formula (III)

$$X_2COOH \qquad (III)$$

[wherein $X_2$ represents the following two groups:

1') a group represented by the general formula:

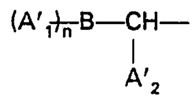

[wherein B and n have the same significance as defined in claim 1 $A'_1$ represents a substituent which is selected from hydroxyl group, a protected hydroxyl group, a lower alkoxy group having 1 to 4 carbon atoms, a halo group, nitro group, a protected amino group, a protected aminomethyl group, and a methylsulfonamide group, and $A'_2$ represents a protected amino group, hydroxyl group, a protected hydroxyl group, carboxyl group, a protected carboxyl group, sulfo group or a protected sulfo group].

2') a group represented by the general formula:

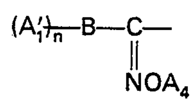

[wherein $A'_1$, B and n have the same significance as defined above and $A_4$ represents hydrogen atom, a lower alkyl group having 1 to 6 carbon atoms, a lower alkenyl group having 2 to 6 carbon atoms, a lower alkynyl group having 2 to 6 carbon atoms, a cycloalkyl group having 3 to 6 carbon atoms or an aryl group, those groups being unsubstituted or substituted with a suitable substituent which is selected from a protected carboxyl group, cyano group, a halo group, carbamoyl group and a lower alkoxycarbonyl group having 1 to 4 carbon atoms], or with reactive derivatives of the carboxylic acid and, thereafter, optionally deprotecting the protecting group in the group $X_2CO$— and/or —$COOR_3$ and, optionally, converting to pharmaceutically acceptable salts thereof.

22. A process for producing optically active compounds of cephalosporin analogs represented by the formula (I')

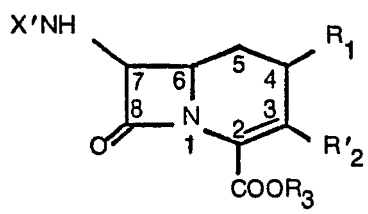

(I')

wherein $R_1$ and $R_3$ have the same significance as defined in claim 1 and $R_2$ represents a halogen atom and X' represents an acyl group represented by the general formula $X_3CO$ wherein $X_3$ represents a group represented by the general formula

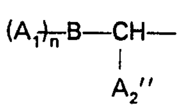

wherein $A_1$ and B have the same significance as defined in claim 1 and $A_2''$ represents amino group, hydroxyl group, carboxyl group or sulfo group and pharmaceutically acceptable salts thereof, which comprises acylating optically inactive compound (II') represented by the formula

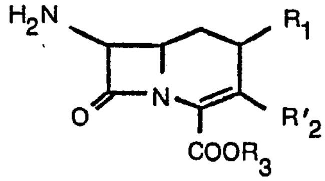

(II')

(wherein $R_1$, $R_2'$ and $R_3$ have the same significance as defined above) or a functionally equivalent compound with carboxylic acid represented by the formula (VIII)

$$X_3COOH \qquad (VIII)$$

(wherein $X_3$ represents the following group represented by the formula

$$(A_1')_n B-\underset{\displaystyle A_2'''}{\underset{|}{CH}}-$$

wherein $A_1'$, B and n have the same significance defined in claim 21, $A_2'''$ represents a protected amino group, a hydroxyl group, a protected hydroxyl group, a carboxyl group, a protected carboxyl group, a sulfo group or a protected sulfo group) or with reactive derivatives of the carboxylic acid and, thereafter, optionally deprotecting the protecting group in the group $X_3CO$ and/or $-COOR_3$, the obtained diastereomeric mixture being separated by conventional method and optionally, converting to pharmaceutically acceptable salts thereof.

23. A process for producing optically active compounds of cephalosporin analogs represented by the general formula (IV)

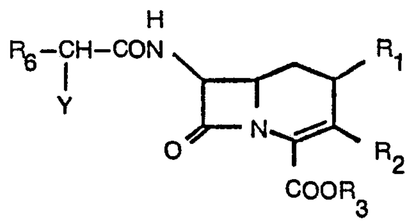

(IV)

(wherein $R_6$ represents a substituted or unsubstituted saturated or unsaturated six-membered carbocyclic or five-membered heterocyclic group wherein the substituent is selected from those specified for $A_1$ in claim 1, $R_1$, $R_2$ and $R_3$ have the same meanings defined in claim 1, Y represents a lower alkyl group having 1 to 5 carbon atoms, a hydroxy group, a carboxy group or an amino group, and the hydrogens at the 6- and 7-positions have cis configuration) and salts thereof, characterized in that $\alpha,\alpha$-disubstituted carboxylic acid represented by the general formula (V)

$$R_6\underset{\displaystyle Y}{\underset{|}{C}}HCOOH \qquad (V)$$

(wherein $R_6$ and Y have the same significance as defined above) or a reactive derivative thereof and an optically inactive compound represented by the general formula (II) or an optically active compound represented by the general formula (II—1)

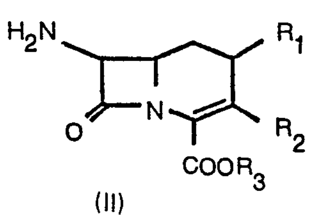

(II)

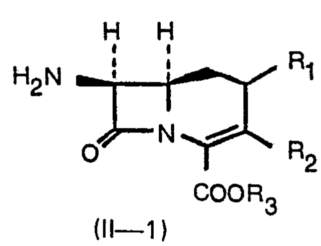

(II—1)

(wherein $R_1$, $R_2$ and $R_3$ have the same significance as defined above) are reacted in the presence of (1) a microorganism having an ability of producing optically active compounds of the cephalosporin analog

represented by the general formula (IV), from the $\alpha,\alpha$-disubstituted carboxylic acid or reactive derivative thereof and the compound represented by the general formula (II) or (II—1) and belonging to the genus *Pseudomonas, Xanthomonas, Escherichia, Aeromonas, Achromobacter, Arthrobacter, Acetobacter, Alcaligenes, Kluyvera, Gluconobacter, Clostridium, Comamonas, Corynebacterium, Sarcina, Straphylococcus, Spirllum, Bacillus, Flavobacterium, Brevibacterium, Protaminobacter, Beneckea, Micrococcus, Proteus, Mycoplana* or *Rhodopseudomonas,* (2) a culture broth of the microorganism, (3) a matter extracted from the culture broth or (4) an enzyme produced by the microorganism and the obtained compound is optionally converted to pharmaceutically acceptable salt by a conventional method.

24. The process according to claim 23 wherein $R_8$ is a phenyl group which is either unsubstituted or p-hydroxy-substituted, and Y is an amino group.

25. The process according to claim 25 wherein $R_1$ is a hydrogen or a methyl group, $R_2$ is a hydrogen or a chlorine atom.

26. Pharmaceutical composition with antimicrobial activity comprising a compound according to any of the claims 1 to 20 together with conventional diluents and/or carriers and/or adjuvants.

**Revendications**

1. Dérivés acylés optiquement actifs de la céphalosporine représentés par la formule générale (I):

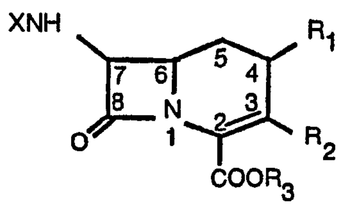

(I)

dans laquelle $R_1$ représente de l'hydrogène ou un groupe alkyle inférieur ayant 1 à 5 atomes de carbone; $R_2$ représente un atome d'hydrogène ou d'halogène; $R_3$ représente de l'hydrogène ou un groupe protecteur d'acide carboxylique; X désigne un groupe acyle représenté par la formule $X_1CO$ où $X_1$ désigne les deux groupes suivants:

1) un groupe représenté par la formule générale:

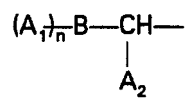

[dans laquelle B représente un carbocycle non saturé à six chaînons qui est choisi parmi les groupes, cyclohexényle, cyclohexadiényle et phényle, ou un hétérocycle à 5 ou 6 chaînons choisi parmi les groupes furyle, thiényle, pyrrolyle, thiazolyle, oxazolyle, isothiazolyle, isoxazolyle, imidazolyle, pyrazolyle, triazolyle, tétrazolyle, pyridinyle, pyrimidinyle, pyrazinyle, pyridinazyle, triazinyle et 5,6-dihydro-1,4-dithi-in-2-yle; $A_1$ représente un ou des substituant(s) choisi(s) parmi les groupes hydroxyle, alcoxy inférieur ayant 1 à 4 atomes de carbone, halogéno, nitro, amino, aminométhyle et méthylsulfamide; n est un nombre allant de 0 à 5 et $A_2$ représente le groupe amino, hydroxyle, carboxyle ou sulfo].

2) un groupe représenté par la formule:

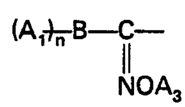

[dans laquelle $A_1$, B et n ont les significations données ci-dessus, et $A_3$ représente l'atome d'hydrogène, un groupe alkyle inférieur ayant 1 à 6 atomes de carbone, un groupe alcényle inférieur ayant 2 à 6 atomes de carbone, un groupe alcynyle inférieur ayant 2 à 6 atomes de carbone, un groupe cycloalkyle ayant 3 à 6 atomes de carbone ou un groupe aryle, ces groupes étant non-substitués ou substitués avec un ou des substituant(s) approprié(s) choisi(s) parmi les groupes carboxyle, cyano, halogéno, carbamoyle et alcoxycarbonyle inférieur ayant 1 à 4 atomes de carbone], et les atomes d'hydrogène sur les positions 6 et 7 ont une configuration cis, et les sels de ces dérivés phrmaceutiquement acceptables.

2. Composé selon la revendication 1, dans lequel $A_1$ est un groupe amino; B est un hétérocycle à 5 chaînons; $A_3$ est un groupe alkyle inférieur ayant 1 à 6 atomes de carbone ou un groupe alkyle inférieur substitué par un groupe carboxyle et le groupe $OA_3$ a une configuration syn.

3. Composé selon la revendication 2, dans lequel $R_2$ est un atome d'hydrogène, B est un groupe thiazolyle substitué sur la position 2, et $A_3$ est un groupe méthyle.

4. Composé selon la revendication 3, dans lequel $R_1$ est un atome d'hydrogène.

5. Composé selon la revendication 4, dans lequel $R_3$ est un atome d'hydrogène, et qui est l'acide (+) - cis - 7 - [2 - (2 - amino - 4 - thiazolyl) - 2 - syn - méthoxy - iminoacétamido] - 1 - azabicyclo[4,2,0]oct - 2 - èn - 8 - one - 2 - carboxylique.

6. Composé selon la revendication 3, dans lequel $R_1$ est le groupe méthyle.

7. Composé selon la revendication 6, dans lequel $R_3$ est un atome d'hydrogène.

8. Composé selon la revendication 7, dans lequel le groupe méthyle sur la position 4 a la même configuration que les atomes d'hydrogène sur les positions 6 et 7, et qui est l'acide (—) - cis - 7$\beta$ - [2 - (2 - amino - 4 - thiazolyl) - 2 - syn - méthoxy - iminoacétamido] - 4$\alpha$ - méthyl - 1 - azabicyclo[4,2,0]oct - 2 - en - 8 - one - 2 - carboxylique.

9. Composé selon la revendication 1, dans lequel $A_1$ est un atome d'hydrogène ou un groupe hydroxyle; B est un groupe phényle, et $A_2$ est un groupe amino.

10. Composé selon la revendication 9, dans lequel $R_1$ et $R_3$ sont des atomes d'hydrogène.

11. Composé selon la revendication 10, dans lequel $R_2$ est un atome de chlore.

12. Composé selon la revendication 11, dans lequel $A_1$ est un atome d'hydrogène et $A_2$ est un groupe amino, et qui est l'acide (6R, 7S) 7 - (R) - phénylglycinamido - 3 - chloro - 1 - azabicyclo-[4,2,0]oct - 2 - èn - 8 - one - 2 - carboxylique.

13. Composé selon la revendication 11, dans lequel $A_1$ est un groupe p-hydroxyle, et $A_2$ est un groupe amino, et que est l'acide (6R, 7S) 7 - (R) - p - hydroxyphénylglycinamido - 3 - chloro - 1 - azabicyclo[4,2,0]oct - 2 - èn - 8 - one - 2 - carboxylique.

14. Composé selon la revendication 9, dans lequel $R_2$ et $R_3$ sont des atomes d'hydrogène.

15. Composé selon la revendication 14, dans lequel $R_1$ est un atome d'hydrogène ou le groupe méthyle.

16. Composé selon la revendication 15, dans lequel $A_1$ est un atome d'hydrogène ou un groupe p-hydroxyle, et $A_2$ est un groupe amino.

17. Composé selon la revendication 16, dans lequel $R_1$ et $A_1$ sont des atomes d'hydrogène, et qui est l'acide (6R, 7S), 7 - (R) - phénylglycinamido - 1 - azabicyclo[4,2,0]oct - 2 - èn - 8 - one - 2 - carboxylique.

18. Composé selon la revendication 16, dans lequel $R_1$ est un atome d'hydrogène, et $A_1$ est un groupe p-hydroxyle, et qui est l'acide (6R, 7S) 7 - (R) - p - hydroxyphénylglycinamido - 1 - azabicyclo[4,2,0]oct - 2 - èn - 8 - one - 2 - carboxylique.

19. Composé selon la revendication 15, dans lequel $R_1$ est le groupe méthyle, et $A_1$ est un atome d'hydrogène, et qui est l'acide (6R, 7S), 7 - (R) - phénylglycinamido - 4 - méthyl - 1 - azabicyclo-[4,2,0]oct - 2 - èn - 8 - one - 2 - carboxylique.

20. Composé selon la revendication 15, dans lequel $R_1$ est le groupe méthyle, et $A_1$ est un groupe p-hydroxyle, et qui est l'acide (6R, 7S) 7 - (R) - p - hydroxyphénylglycinamido - 4 - méthyl - 1 - azabicyclo[4,2,0]oct - 2 - èn - 8 - one - 2 - carboxylique.

21. Procédé de préparation de dérivés de la céphalosporine représentés par la formule générale (I) selon la revendication 1, et de leurs sels pharmaceutiquement acceptables, qui consiste à acyler des dérivés optiquement actifs de la céphalosporine représentés par la formule générale (II):

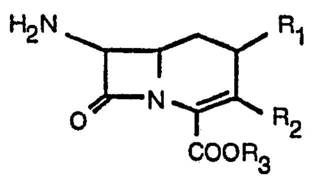

(II)

(dans laquelle $R_1$, $R_2$ et $R_3$ ont les signification données ci-dessus, et les atomes d'hydrogène sur les positions 6 et 7 ont la configuration "cis"), ou un composé fonctionnellement équivalent, avec un acide carboxylique représenté par la formule générale (III):

$$X_2COOH \qquad (III)$$

dans laquelle $X_2$ représente les deux groupes suivants:

1') un groupe représenté par la formule générale:

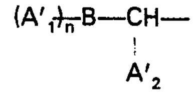

[dans laquelle B et n ont les significations indiquées dans la revendication 1; $A'_1$ représente un substituant choisi parmi un groupe hydroxyle, un groupe hydroxyle protégé, les groupes alcoxy inférieur ayant 1 à 4 atomes de carbone, halogéno, nitro, amino protégé, aminométhyle protégé et méthylsulfamide; et $A'_2$ représente un groupe amino protégé, hydroxyle, hydroxyle protégé, carboxyle, carboxyle protégé, sulfo ou sulfo protégé].

2') un groupe représenté par la formule générale:

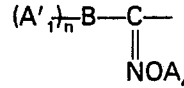

[dans laquelle $A'_1$, B et n ont les significations données ci-dessus, et $A_4$ représente un atome d'hydrogène, un groupe alkyle inférieur ayant 1 à 6 atomes de carbone, un groupe alcényle inférieur ayant 2 à 6 atomes de carbone, un groupe alcynyle inférieur ayant 2 à 6 atomes de carbone, un groupe cycloalkyle ayant 3 à 6 atomes de carbone ou un groupe aryle, ces groupes étant non substitués ou substitués avec un substituant approprié choisi parmi les groupes carboxyle protégé, cyano, halogéno, carbamoyle, et alcoxycarbonyle inférieur ayant 1 à 4 atomes de carbone], ou avec des dérivés réactifs de l'acide carboxylique, puis éventuellement à éliminer le groupe protecteur dans le groupe $X_2CO$— et/ou —$COOR_3$, et, éventuellement, à convertir les dérivés en question en leurs sels pharmaceutiquement acceptables.

22. Procédé de préparation de composés optiquement actifs de dérivés de la céphalosporine représentés par la formule (I'):

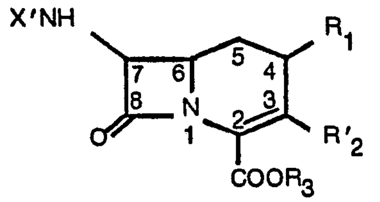

(I')

dans laquelle $R_1$ et $R_3$ ont les significations définies dans la revendication 1, et $R'_2$ représente un atome d'halogène, et X' désigne un groupe acyle représenté par la formule générale $X_3CO$ où $X_3$ est un groupe représenté par la formule générale:

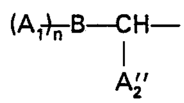

dans laquelle $A_1$ et B ont les significations définies dans la revendication 1, et $A_2''$ représente un groupe amino, hydroxyle, carboxyle ou sulfo, et de leurs sels pharmaceutiquement acceptables, qui consiste à acyler un composé optiquement inactif (II') représenté par la formule:

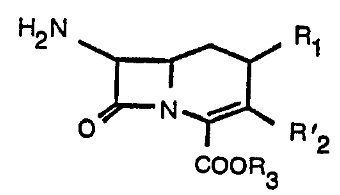

(II')

(dans laquelle $R_1$, $R'_2$ et $R_3$ ont les significations données ci-dessus), ou un composé fonctionnellement équivalent, avec un acide carboxylique représenté par la formule (VIII):

$$X_3COOH \qquad (VIII)$$

(dans laquelle $X_3$ représenté le groupe suivant ayant la formula:

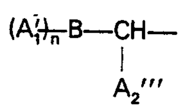

dans laquelle $A'_1$, B et n ont les significations données dans la revendication 21; $A_2'''$ représente un groupe amino protégé hydroxyle, hydroxyle protégé, carboxyle, carboxyle protégé, sulfo ou sulfo protégé), ou avec des dérivés réactifs de l'acide carboxylique, puis, éventuellement à éliminer le groupe protecteur dans le groupe $X_3CO$— et/ou —$COOR_3$, le mélange de diastéréoisomères obtenu étant séparé par un procédé classique et, éventuellement, à convertir les composés en question en leurs sels pharmaceutiquement acceptables.

23. Procédé de préparation de composés optiquement actifs de dérivés de la céphalosporine représentés par la formule générale (IV):

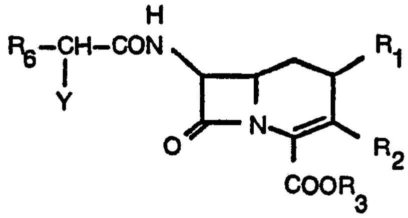

(IV)

(dans laquelle $R_6$ représente un groupe carbocyclique à six chaîons, saturé ou non saturé, substitué ou non substitué, ou bien un groupe hétérocyclique à cinq chaînons où le substituant est choisi parmi ceux indiqués pour $A_1$ dans la revendication 1; $R_1$, $R_2$ et $R_3$ ont les significations définies dans la revendication 1; Y représente un groupe alkyle inférieur ayant 1 à 5 atomes de carbone, hydroxyle, carboxyle ou amino, et les hydrogènes sur les positions 6 et 7 ont la configuration "cis") et de leurs sels, caractérisé par le fait qu'on fait réagir l'acide carboxylique, $\alpha,\alpha$-disubstitué représenté par la formule (V):

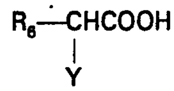

(V)

(dans laquelle $R_6$ et Y ont les significations données ci-dessus), ou un de ses dérivés réactifs, et un composé optiquement inactif représenté par la formule (II), ou un composé optiquement actif représenté par la formule (II—1):

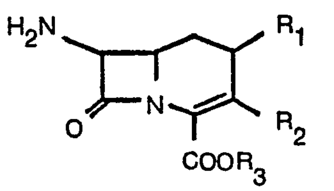

(II)

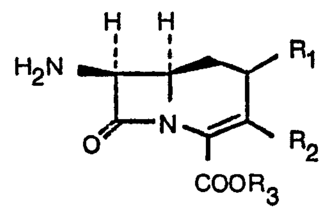

(II—1)

(dans lesquelles $R_1$, $R_2$ et $R_3$ ont les significations données ci-dessus), en présence de (1) un microorganisme capable de produire des composés optiquement actifs du dérivé de la céphalosporine représenté par la formule générale (IV), à partir de l'acide carboxylique $\alpha,\alpha$-disubstitué, ou d'un de ses dérivés réactifs, et du composé représenté par la formule générale (II) ou (II—1), et appartenant au genre *Pseudomonas, Xanthomonas, Escherichia, Aeromonas, Achromobacter, Arthrobacter, Acetobacter, Alcaligenes, Kluyvera, Gluconobacter, Clostridium, Comamonas, Corynebacterium, Sarcina, Staphylococcus, Spirillum, Bacillus, Flavobacterium, Brévibactérium, Protaminobacter, Beneckea, Micrococcus, Proteus, Mycoplana* ou *Rhodopseudomonas*. (2) un bouillon de culture du microorganisme, (3) une matière extraite du bouillon de culture ou (4) une enzyme produite par le microorganisme, et qu'on convertit éventuellement le composé obtenu en son sel pharmaceutiquement acceptable par un procédé classique.

24. Procédé selon la revendication 23, où $R_6$ est un groupe phényle, soit non substitué, soit substitué par un groupe p-hydroxyle, et Y est un groupe amino.

25. Procédé selon la revendication 24, où $R_1$ est un atome d'hydrogène ou le groupe méthyle; $R_2$ est un atome d'hydrogène ou de chlore.

26. Compositions pharmaceutiques possédant une activité antimicrobienne comprenant un composé selon l'une quelconque des revendications 1 à 20, en même temps que des diluants et-ou des véhicules et-ou des adjuvants classiques.

**Patentansprüche**

1. Optisch aktive acylierte cephalosporinähnliche Verbindungen der allgemeinen Formel I

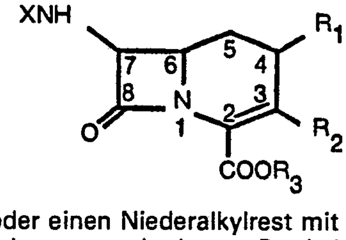

(I)

in der $R_1$ ein Wasserstoffatom oder einen Niederalkylrest mit 1 bis 5 Kohlenstoffatomen bedeutet, $R_2$ ein Wasserstoffatom oder ein Halogenatom bedeutet, $R_3$ ein Wasserstoffatom oder eine Carbonsäure-Schutzgruppe darstellt, X eine Acylgruppe der Formel $X_1CO$ bedeutet, in der $X_1$ die folgenden zwei Gruppen darstellt:

1) Eine Gruppe der allgemeinen Formel

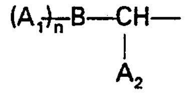

[worin B einen ungesättigten 6 gliedrigen Carbozyklus bedeutet, aus der Gruppe Cyclohexenyl- Cyclohexadienyl- und Phenylgruppe, oder einen 5- oder 6 gliedriger Heterozyklus, aus der Gruppe Furyl-, Thienyl-, Pyrrolyl-, Thiazolyl-, Oxazolyl-, Isothiazolyl-, Isoxazolyl-, Imidazolyl-, Pyrazolyl-, Triazolyl-, Tetrazolyl-, Pyridinyl-, Pyrimidinyl-, Pyrazinyl-, Pyridazinyl-, Triazinyl- und 5,6-Dihydro-1,4-dithiin-2-yl-Gruppe bedeutet, $A_1$ einen oder mehrere Substituenten bedeutet, aus der Gruppe Hydroxylgruppe, niederer Alkoxyrest mit 1 bis 4 Kohlenstoffatomen, Halogenatom, Nitrogruppe, Aminogruppe, Aminomethylgruppe und Methylsulfonamidgruppe, n eine Zahl von 0 bis 5 ist und $A_2$ eine Aminogruppe, Hydroxylgruppe, Carboxylgruppe oder Sulfogruppe bedeutet],

2) eine Gruppe der allgemeinen Formel

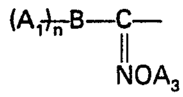

[worin $A_1$, B und n die vorstehende Bedeutung haben und $A_3$ ein Wasserstoffatom, einen niederen Alkylrest mit 1 bis 6 Kohlenstoffatomen, eine niedere Alkenylgruppe mit 2 bis 6 Kohlenstoffatomen, eine niedere Alkinylgruppe mit 2 bis 6 Kohlenstoffatomen, eine Cycloalkylgruppe mit 3 bis 6 Kohlenstoffatomen oder eine Arylgruppe bedeutet, wobei diese Gruppen unsubstituiert oder mit einem oder mehreren geeigneten Substituenten versehen sind aus der Gruppe Carboxylrest, Cyanogruppe, Halogenatom, Carbamoylgruppe und niedere Alkoxycarbonylgruppe mit 1 bis 4 Kohlenstoffatomen], und die Wasserstoffatome in den 6- und 7-Stellungen die cis-Konfiguration haben, sowie ihre pharmazeutisch verträglichen Salze.

2. Verbindung nach Anspruch 1, dadurch gekennzeichnet, daß $A_1$ eine Aminogruppe, B einen 5 gliedrigen Heterozyklus, $A_3$ eine niedere Alkylgruppe mit 1 bis 6 Kohlenstoffatomen oder eine substituierte niedere Alkylgruppe mit einer Carboxylgruppe als Substituenten bedeutet, und der $OA_3$-Rest die syn-Konfiguration hat.

3. Verbindung nach Anspruch 2, dadurch gekennzeichnet, daß $R_2$ ein Wasserstoffatom bedeutet, B eine 2-substituierte Thiazolylgruppe und $A_3$ eine Methylgruppe bedeutet.

4. Verbindung nach Anspruch 3, dadurch gekennzeichnet, daß $R_1$ ein Wasserstoffatom bedeutet.

5. Verbindung nach Anspruch 4, in der $R_3$ ein Wasserstoffatomen bedeutet, nämlich (+) - cis - 7 - [2 - (2 - Amino - 4 - thiazolyl) - 2 - syn - methoxyiminoacetamido] - 1 - azabicyclo[4.2.0]oct - 2 - en - 8 - on - 2 - carbonsäure.

6. Verbindung nach Anspruch 3, dadurch gekennzeichnet, daß $R_1$ eine Methylgruppe bedeutet.

7. Verbindung nach Anspruch 6, dadurch gekennzeichnet, daß $R_3$ ein Wasserstoffatom bedeutet.

8. Verbindung nach Anspruch 7, in der die Methylgruppe in der 4-Stellung die gleiche Konfiguration aufweist, wie die Wasserstoffatome in den 6- und 7-Stellungen, nämlich (—) - cis - 7$\beta$ - [2 - (2 - Amino - 4 - thiazolyl) - 2 - syn - methoxyiminoacetamido] - 4 - $\alpha$ - methyl - 1 - azabicyclo[4.2.0]oct - 2 - en - 8 - on - 2 - carbonsäure.

9. Verbindung nach Anspruch 1, dadurch gekennzeichnet, daß $A_1$ ein Wasserstoffatom oder eine Hydroxylgruppe bedeutet, B eine Phenylgruppe und $A_2$ eine Aminogruppe darstellt.

10. Verbindung nach Anspruch 9, dadurch gekennzeichnet, daß $R_1$ und $R_3$ Wasserstoffatome bedeuten.

11. Verbindung nach Anspruch 10, dadurch gekennzeichnet, daß $R_2$ ein Chloratom bedeutet.

12. Verbindung nach Anspruch 11, in der $A_1$ ein Wasserstoffatom und $A_2$ eine Aminogruppe bedeutet, nämlich (6R, 7S) - 7 - (R) - Phenylglycinamido - 3 - chlor - 1 - azabicyclo[4.2.0]oct - 2 - en - 8 on - 2 - carbonsäure.

13. Verbindung nach Anspruch 11, in der $A_1$ eine p-Hydroxylgruppe und $A_2$ eine Aminogruppe bedeutet, nämlich (6R, 7S) - 7 - (R) - p - Hydroxyphenylglycinamido - 3 - chlor - 1 - azabicyclo[4.2.0]-oct - 2 - en - 8 - on - 2 - carbonsäure.

14. Verbindung nach Anspruch 9, dadurch gekennzeichnet, daß $R_2$ und $R_3$ Wasserstoffatome bedeuten.

15. Verbindung nach Anspruch 14, dadurch gekennzeichnet, daß $R_1$ ein Wasserstoffatom oder eine Methylgruppe bedeutet.

16. Verbindung nach Anspruch 15, dadurch gekennzeichnet, daß $A_1$ ein Wasserstoffatom oder eine p-Hydroxylgruppe bedeutet und $A_2$ eine Aminogruppe darstellt.

17. Verbindung nach Anspruch 16, in der $R_1$ und $A_1$ Wasserstoffatome bedeuten, nämlich (6R,

7S) - 7 - (R) - Phenylglycinamido - 1 - azabicyclo[4.2.0]oct - 2 - en - 8 - on - 2 - carbonsäure.

18. Verbindung nach Anspruch 16, in der $R_1$ ein Wasserstoffatom und $A_1$ eine p-Hydroxylgruppe bedeutet, nämlich (6R, 7S) - 7 - (R) - p - Hydroxyphenylglycinamido - 1 - azabicyclo[4.2.0]oct - 2 - en - 8 - on - 2 - carbonsäure.

19. Verbindung nach Anspruch 15, in der $R_1$ eine Methylgruppe und $A_1$ ein Wasserstoffatom bedeutet, nämlich (6R, 7S) - 7 - (R) - Phenylglycinamido - 4 - methyl - 1 - azabicyclo[4.2.0]oct - 2 - en - 8 - on - 2 - carbonsäure.

20. Verbindung nach Anspruch 15, in der $R_1$ eine Methylgruppe und $A_1$ eine p-Hydroxylgruppe bedeutet, nämlich (6R, 7S) - 7 - (R) - p - Hydroxyphenylglycinamido - 4 - methyl - 1 - azabicyclo-[4.2.0]oct - 2 - en - 8 - on - 2 - carbonsäure.

21. Verfahren zur Herstellung von cephalosporinähnlichen Verbindungen der allgemeinen Formel I nach Anspruch 1 und ihrer pharmazeutisch verträglichen Salze, dadurch gekennzeichnet, daß man optisch aktive Cephalosporinan-Analoga der allgemeinen Formel II:

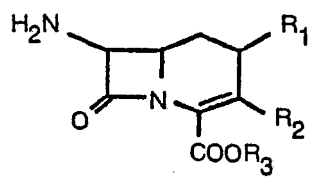

(II)

(in der $R_1$, $R_2$ und $R_3$ die vorstehende Bedeutung haben und die Wasserstoffatome in den 6- und 7-Stellungen die "cis"-Konfiguration haben) oder eine funktional äquivalente Verbindung mit einer Carbonsaüre der allgemeinen Formel III acyliert

$$X_2COOH \qquad (III)$$

in der $X_2$ die folgenden beiden Gruppen bedeutet:

1') eine Gruppe der allgemeinen Formel

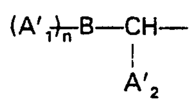

[in der B und n die gleiche Bedeutung wie in Anspruch 1 haben, $A'_1$ einen Substituenten aus der Gruppe Hydroxylgruppe, geschützte Hydroxylgruppe, niedere Alkoxygruppe mit 1 bis 4 Kohlenstoffatomen, Halogenatom, Nitrogruppe, geschützte Aminogruppe, geschützte Aminomethylgruppe und Methylsulfonamidogruppe und $A'_2$ eine geschützte Aminogruppe, eine Hydroxylgruppe, eine geschützte Hydroxylgruppe, eine Carboxylgruppe, eine geschützte Carboxylgruppe, eine Sulfogruppe oder eine geschützte Sulfogruppe bedeutet]

2') eine Gruppe der allgemeinen Formel:

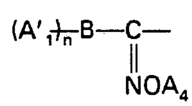

[in der $A'_1$, B und n die vorstehende Bedeutung haben und $A_4$ ein Wasserstoffatom, eine niedere Alkylgruppe mit 1 bis 6 Kohlenstoffatomen, eine niedere Alkenylgruppe mit 2 bis 6 Kohlenstoffatomen, eine niedere Alkinylgruppe mit 2 bis 6 Kohlenstoffatomen, eine Cycloalkylgruppe mit 3 bis 6 Kohlenstoffatomen oder eine Arylgruppe bedeutet, wobei diese Gruppen unsubstituiert oder mit einem geeigneten Substituenten aus der Gruppe geschützte Carboxylgruppe, Cyanogruppe, Halogenatom, Carbamoylgruppe und niedere Alkoxycarbonylgruppe mit 1 bis 4 Kohlenstoffatomen substituiert sind], oder mit reaktiven Derivaten der Carbonsäure, und man anschließend gegebenenfalls die Schutzgruppe in den Resten $X_2CO$— und/oder —$COOR_3$ entfernt, und gegebenenfalls die entstandenen Verbindungen in die pharmazeutisch verträglichen Salze umwandelt.

22. Verfahren zur Herstellung optisch aktiver Verbindungen von Cephalosporin-Analoga der Formel I'

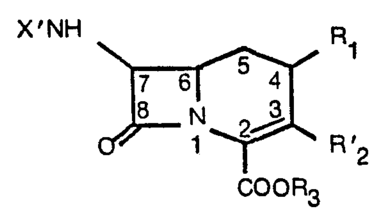

(I')

in der $R_1$ und $R_3$ die in Anspruch 1 angegebene Bedeutung haben und $R'_2$ ein Halogenatom bedeutet, X' eine Acylgruppe der allgemeinen Formel $X_3CO$ darstellt, in der $X_3$ eine Gruppe der allgemeinen Formel

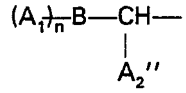

bedeutet, in der $A_1$ und B die in Anspruch 1 angegebene Bedeutung haben und $A_2''$ eine Aminogruppe, Hydroxylgruppe, Carboxylgruppe oder Sulfogruppe bedeutet, sowie ihre pharmazeutisch verträglichen Salze, dadurch gekennzeichnet, daß man eine optisch inaktive Verbindung der Formel II'

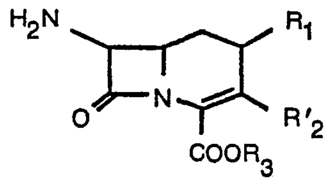

(II')

[in der $R_1$, $R_2'$ und $R_3$ die vorstehende Bedeutung haben], oder eine funktional äquivalente Verbindung mit einer Carbonsäure der Formel VIII acyliert:

$$X_3COOH \quad \text{(VIII)}$$

[in der $X_3$ die Gruppe der nachstehenden Formel bedeutet

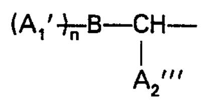

in der $A_1'$, B und n die in Anspruch 21 angegebene Bedeutung haben, $A_2'''$ eine geschützte Aminogruppe, eine Hydroxylgruppe, eine geschützte Hydroxylgruppe, eine Carboxylgruppe, eine geschützte Carboxylgruppe, eine Sulfogruppe oder eine geschützte Sulfogruppe bedeutet], oder mit einem reaktiven Derivat der Carbonsäure, und man anschließend gegebenenfalls die Schutzgruppe in den Resten $X_3CO$ und/oder —$COOR_3$ abspaltet, das erhaltene diastereomere Gemisch in an sich bekannter Weise auftrennt, und man gegebenenfalls die erhaltene Verbindung in ihre pharmazeutisch verträglichen Salze umwandelt.

23. Verfahren zur Herstellung optisch aktiver Verbindungen von Cephalosporin-Analoga der allgemeinen Formel IV

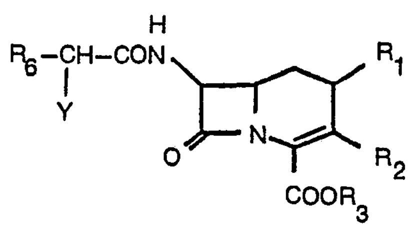

(IV)

[in der $R_6$ einen substituierten oder unsubstituierten gesättigten oder ungesättigten 6 gliedrigen carbocyclischen oder 5 gliedrigen heterocyclischen Rest bedeutet, in dem der Substituent aus der Gruppe der Substituenten für den Rest $A_1$ in Anspruch 1 ausgewählt ist, $R_1$, $R_2$ und $R_3$ die in Anspruch 1 angegebene Bedeutung haben, Y eine niedere Alkylgruppe mit 1 bis 5 Kohlenstoffatomen, eine Hydroxylgruppe, eine Carboxygruppe oder eine Aminogruppe bedeutet, und die Wasserstoffatome in den 6- und 7-Stellungen die cis-Konfiguration haben] und ihre Salze, dadurch gekennzeichnet, daß man eine $\alpha,\alpha$-disubstituierte Carbonsäure der allgemeinen Formel V

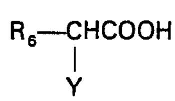

(V)

[in der $R_6$ und Y die vorstehende Bedeutung haben], oder ihr reaktives Derivat sowie eine optisch inaktive Verbindung der allgemeinen Formel II oder eine optisch aktive Verbindung der allgemeinen Formel II—1

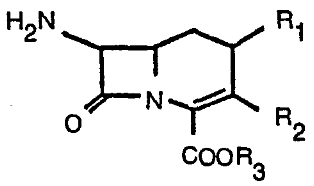

(II)

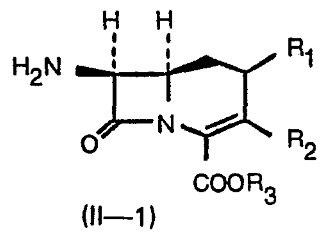

(II—1)

[in denen $R_1$, $R_2$ und $R_3$ die vorstehende Bedeutung haben], zur Umsetzung bringt, in Gegenwart von

1) einem Mikroorganismus, der die Fähigkeit zur Herstellung optisch aktiver Verbindungen der Cephalosporin-Analoga der allgemeinen Formel IV aufweist, ausgehend von einer $\alpha,\alpha$-disubstituierten Carbonsäure oder ihren reaktiven Derivat und der Verbindung der allgemeinen Formel II oder II—1, und der zur Gattung *Pseudomonas, Xanthomonas, Escherichia, Aeromonas, Achromobacter, Arthrobacter, Acetobacter, Alcaligenes, Kluyvera, Gluconobacter, Clostridium, Comamonas, Corynebacterium, Sarcina, Staphylococcus, Spirillum, Bacillus, Flavobacterium, Brevibacterium, Protaminobacter, Beneckea, Micrococcus, Proteus, Mycoplana* oder *Rhodopseudomonas* gehört,

2) einer Kulturflüssigkeit des Mikroorganismus,

3) einem Extrakt aus der Kulturflüssigkeit, oder

4) einem durch den Mikroorganismus produzierten Enzym,

und man die erhaltene Verbindung gegebenenfalls in an sich bekannter Weise in ihr pharmazeutisch verträgliches Salz umwandelt.

24. Verfahren nach Anspruch 23, dadurch gekennzeichnet, daß $R_6$ eine Phenylgruppe bedeutet, die entweder unsubstituiert oder p-hydroxysubstituiert ist und Y eine Aminogruppe bedeutet.

25. Verfahren nach Anspruch 24, dadurch gekennzeichnet, daß $R_1$ eine Wasserstoffatom oder eine Methylgruppe bedeutet und $R_2$ ein Wasserstoffatom oder ein Chloratom bedeutet.

26. Pharmazeutische Zubereitungen mit antimikrobieller Wirksamkeit, enthaltend eine Verbindung nach jedem der Ansprüche 1 bis 20 sowie übliche Verdünnungsmittel und/oder Trägerstoffe und/oder Hilfsstoffe.